# EUROPÄISCHE PATENTSCHRIFT

(11) **EP 2 084 280 B1**
(45) Veröffentlichungstag und Bekanntmachung des Hinweises auf die Patenterteilung: **18.04.2012**
(21) Anmeldenummer: 07819374.5
(22) Anmeldetag: 26.10.2007
(51) Int. Cl.: C12N 15/10

(54) **INTEGRIERTES MIKROFLUIDISCHES BAUTEIL ZUM AUFREINIGEN VON ANALYTMOLEKÜLEN SOWIE VERFAHREN ZUM AUFREINIGEN**
INTEGRATED MICROFLUIDIC COMPONENT FOR PURIFYING ANALYTE MOLECULES AND PURIFICATION METHOD
DISPOSITIF MICROFLUIDIQUE INTÉGRÉ POUR LA PURIFICATION DE MOLÉCULES D'ANALYTE ET PROCÉDÉ DE PURIFICATION

(30) Priorität: 27.10.2006 DE 102006050871
(43) Veröffentlichungstag der Anmeldung: 05.08.2009
(73) Patentinhaber: Albert-Ludwigs-Universität Freiburg, 79085 Freiburg (DE)
(72) Erfinder: VULTO, Paul, 79117 Freiburg (DE); URBAN, Gerald, 79104 Freiburg (DE)
(74) Vertreter: reuteler & cie SA
(86) Internationale Anmeldenummer: PCT/EP2007/009330
(87) Internationale Veröffentlichungsnummer: WO 2008/049638

(56) Entgegenhaltungen:
- WO-A-97/41219
- WO-A-2004/013329
- DE-U1- 29 702 254
- US-A1- 2006 088 867
- SCHÖNHUBER WILHELM ET AL: "Utilization of tmRNA sequences for bacterial identification" BMC MICROBIOLOGY, BIOMED CENTRAL, LONDON, GB, Bd. 1, Nr. 1, 7. September 2001 (2001-09-07), Seite 20, XP021014766 ISSN: 1471-2180

## Beschreibung

Die vorliegende Erfindung bezieht sich auf ein integriertes mikrofluidisches Bauteil zum Aufreinigen von Analytmolekülen und insbesondere auf ein derartiges Bauteil, bei dem eine Separationsstrecke zum Auftrennen von Analytmolekülen und anderen Bestandteilen einer Probe verwendet wird, und bei dem mindestens eine Probenkammer zum Aufnehmen einer die Analytmoleküle enthaltenden Probe und mindestens eine Sammelkammer zum Aufnehmen der aufgereinigten Analytmoleküle vorgesehen ist.

Derartige mikrofluidische Bauteile, die auch oft als Mikrochip oder als mikrofluidischer Chip bezeichnet werden, sind beispielsweise für die DNA-Extraktion aus der internationalen Patentanmeldung WO 2004/013329 A1 bekannt. Die DNA-Extraktionsvorrichtung gemäß dieser Druckschrift beinhaltet einen Hauptkanal mit einem ersten Probenstrom, in dem das DNA enthaltende Material vorliegt, typisch Bakterienzellen oder Zellbruchstücke. Das DNA enthaltende Material wird zunächst einem Elektroporationsschritt unterworfen, um die DNA freizusetzen, und anschließend wird die DNA in einen zweiten Parallelstrom eines zweiten Fluids eingeführt, um mittels Elektroseparation von den unerwünschten Bestandteilen getrennt zu werden. Allerdings können mit diesem bekannten Verfahren nur negativ geladene Teilchen von positiv geladenen oder neutralen Komponenten getrennt werden.

Weiterhin existieren makroskopische Auftrennungsverfahren zum Aufreinigen von Nukleinsäuren, die auf dem Prinzip der Elektrophorese basieren. Beispiele für derartige Auftrennungsverfahren sind in der kanadischen Offenlegungsschrift CA 2318973 A1, der veröffentlichten europäischen Patentanmeldung EP 03 824 246 A2, der kanadischen Patentschrift CA 2130751 C, der US-amerikanischen Patentschrift 5,384,022 sowie der US-amerikanischen Patentanmeldung 2002/0168643 A1 dargestellt.

Die Druckschrift CA 2318973 A1 zeigt verschiedene Verfahren zum Aufreinigen von Nukleinsäuren unter Verwendung von Gelelektrophorese in einer Probe, ohne jedoch einen Detektionsschritt oder einen integrierten Lyseschritt zu erwähnen.

Die EP 03 824 246 A2 beschreibt ein kontinuierliches Durchfluss-Elektrophoresesystem, das in der präparativen Aufreinigung von Nukleinsäuren auf der Basis einer Gelelution eingesetzt werden kann. Das System ist hocheffiziente Aufreinigung von ähnlichen Komponenten wie DNA-Stücken unterschiedlicher Länge konzipiert und legt weder ein integriertes Affinitätsexperiment, noch Probenaufbereitungsschritte, wie beispielsweise eine Lyse, nahe.

Weiterhin ist aus der CA 2130751 C eine Anordnung bekannt, mit deren Hilfe nach einer konventionellen Elektrophorese die einzelnen Komponenten aus dem Gel eluiert werden können. Dabei konzentriert sich die Anordnung auf die Gewinnung von Material, das einem ersten Analyseschritt unterworfen wurde, um es einer nachfolgenden weiteren Analyse zu unterwerfen. Diese Druckschrift befasst sich aber weder mit einer integrierten Vorbehandlung noch mit einem integrierten Nachweis.

Aus dem US Patent 5,384,022 ist ebenfalls eine Vorrichtung zum Extrahieren einer DNA-Bande zur weiteren Analyse bekannt, wobei aber keine integrierte Analyseeinrichtung nahegelegt wird.

Die US-amerikanische Patentanmeldung 2002/0168643 A1 schließlich zeigt eine ähnliche Anordnung zur ultra-reinen Aufreinigung, die eine parallele Durchführung von mehreren Experimenten erlaubt, aber keinen integrierten Nachweis ermöglicht.

Eine miniaturisierte Vorrichtung zur Aufreinigung von Nukleinsäuren unter Verwendung von Mikrostrukturen aus Silizium ist in Nathaniel C. Cady et al.: "Nucleic acid purification using microfabricated silicon structures" Biosensors and Bioelectronics, 19 (2003) 59-66, beschrieben. Hier werden mikrostrukturierte Säulengeometrien in einem Durchflusskanal zur On-Chip-Aufreinigung von DNA vor einem PCR-Schritt verwendet. Allerdings ist dieses Verfahren für eine unmittelbare Detektion der DNA-Moleküle nicht empfindlich genug, so dass immer eine Amplifikation über einen PCR-Schritt erforderlich ist. Weiterhin hat die hier gezeigte Anordnung den Nachteil, dass ein komplexes Aktuierungsschema durch externe Pumpensysteme benötigt wird und dass das Aufreinigungsverfahren aus einer Vielzahl von Schritten, wie einer Adsorption, einem Waschschritt und einem Desorptionsschritt besteht.

Weiterhin existieren verschiedene Veröffentlichungen, die ein Sammeln von Fraktionen nach einer elektrophoretischen Separation zeigen: R. Lin et al.: "Selective extraction of sizefractioned DNA samples in microfabricated electrophoresis devices", J. Chromatography A, 1010 (2003), 255-268, und J. Khandurina et al.: "Micropreparative fraction collection in microfluidic devices", Anal. Chem., 2002, 74, 1737-1740. Das Ziel dieser Veröffentlichungen war aber, die konventionelle Elektrophorese auf einem Chip zu zeigen und nachfolgend die Extraktion der spezifischen Separationsbande für eine weitere Analyse durchzuführen. Eine Aufreinigung im eigentlichen Sinne erfolgte hier nicht. Weiterhin wurden die Fraktionen nach dem Trennen ähnlicher Komponenten gesammelt, beispielsweise Bandenseparation von DNA, was letztendlich nur eine Miniaturisierung von makrobiologischen Standardprotokollen darstellt.

Die WO 2006/071770 A2 offenbart ein Extraktionsverfahren für genomische DNA, das auf einer Festphasenextraktion basiert. Als Festphasenträger werden gemäß dieser Druckschrift magnetische Perlen und Filter verwendet, wie dies als klassische Aufreinigungsmethode bekannt ist. Nach der Aufreinigung wird ein Teil der genomische DNA unter Verwendung einer PCR amplifiziert. Anschließend wird das amplifizierte PCR-Produkt mittels Elektrophorese von den Primern getrennt.

*"D1 beschreibt Verfahren und Vorrichtungen zur Reinigung von Zielmolekülen (z.B. Nukleinsäuren). Eine Separationsregion, die eine elektrophoretische Matrix mit immobilisierten Capture Probes enthält, hält die Zielmoleküle zurück, während das unerwünschte Material die Separationsregion durchläuft. Das elektrophoretische Medium wird dann in einem weiteren Schritt behandelt um den ausgebildeten Komplex zwischen Zielmolekülen und Capture Probe aufzulösen und die Zielmoleküle freizugeben.*

*D2 beschreibt ein Verfahren zur Aufarbeitung von Nukleinsäuren oder Mischungen von Nukleinsäuren aus Zellbruchstücken oder anderen Biomolekül-Mischungen. Das Verfahren aus D2 beinhaltet einen Schritt, wobei eine Elektrode in Kontakt mit der Nukleinsäure-Mischung gebracht wird, -wobei eine Spannung angelegt ist, die die Nukleinsäuren anzieht- und einen Schritt worin die Elektrode, an der die Nukleinsäuren gebunden sind, aus der Nukleinsäure-Mischung entfernt wird."*

Daher besteht die Aufgabe, die der vorliegenden Erfindung zugrunde liegt, darin, ein integriertes mikrofluidisches Bauteil zum Aufreinigen von Analytmolekülen anzugeben, das eine schnelle, empfindliche und automatisierbare Aufreinigung und Bestimmung von Analytmolekülen, beispielsweise Nukleinsäuren, ermöglicht.

Diese Aufgabe wird durch den Gegenstand der unabhängigen Patentansprüche gelöst. Vorteilhafte Weiterbildungen sind Gegenstand der abhängigen Patentansprüche.

Dabei basiert die vorliegende Erfindung auf der Idee, zur Aufreinigung des Analyten eine mikrofluidische, elektrophoretische Gelfiltrationsstrecke zu verwenden. Neben einer hohen Effizienz und Analytausbeute bietet das erfindungsgemäße integrierte mikrofluidische Bauteil insbesondere im Zusammenhang mit dem Nachweis von Ribonukleinsäure, RNA, beispielsweise Transfer-Messenger-RNA, tmRNA, den Vorteil, dass durch die schnelle On-Chip-Aufreinigung unter im Wesentlichen RNase-freien Bedingungen eine Degradation der empfindlichen RNA durch dieses Enzym weitestgehend vermieden werden kann.

Gemäß einer vorteilhaften Ausführungsform der vorliegenden Erfindung ist die Separationsstrecke durch eine elektrophoretische Gelfiltrationsstrecke gebildet. Beispielsweise kann ein 4 %iges NuSieve-Gel verwendet werden. Alternative Gelmaterialien sind Agarose-Gele, Polyacrylamid-Gele, oder eine Mischung dieser beiden. Die Gele können in denaturierender Form, d.h. mit Beimengungen von Formaldehyd, Glycerin, DMSO oder Harnstoff eingesetzt werden, müssen aber nicht zwangsläufig so behandelt sein.

Insbesondere ist vorliegend ein Integriertes fluidisches Bauteil, wobei die elektrophoretische Gelfiltrationsstrecke mindestens einen Seitenkanal aufweist, der durch mindestens eine zusätzliche Elektrode aktuierbar ist.

Insbesondere ist auch vorliegend ein Integriertes fluidisches Bauteil, wobei die elektrophoretische Gelfiltrationsstrecke mindestens einen Seitenkanal aufweist, der durch mindestens eine zusätzliche Elektrode aktuierbar ist und wobei der mindestens eine Seitenkanal so ausgebildet ist, dass er für eine Vorabtrennung von unerwünschten Probenbestandteilen, die eine höhere Migrationsgeschwindigkeit haben als die Analytmoleküle, betreibbar ist.

Insbesondere ist auch vorliegend ein Integriertes fluidisches Bauteil, wobei die elektrophoretische Gelfiltrationsstrecke mindestens einen Seitenkanal aufweist, der durch mindestens eine zusätzliche Elektrode aktuierbar ist, wobei der mindestens eine Seitenkanal betreibbar ist, um eine Sequenz von Gelen unabhängig voneinander zu aktuieren.

Bei Anlegen einer Spannung zwischen Anfang und Ende der Gelfiltrationsstrecke wird die RNA nach dem bekannten Elektrophoreseprinzip von den übrigen Probenbestandteilen getrennt. Dabei basiert die Elektrophorese auf einer Kombination von Grösse, Gestalt und Ladung der zu trennenden Komponenten. Nukleinsäuren haben beispielsweise eine relativ hohe Ladung im Vergleich zu den meisten Proteinen. Ihre Form ist eher länglich im Gegensatz zu den komplexen dreidimensionalen Formen der meisten Proteine. Daher besitzt RNA eine höhere Wanderungsgeschwindigkeit als die meisten anderen Proteine in einer elektrophoretischen Separationsmatrix. Weiterhin weisen Proteine wie dies allgemein bekannt ist, entsprechend ihrem isoelektrischen Punkt unter bestimmten Umgebungsbedingungen eine positive oder negative Ladung auf. Durch eine geschickte Wahl der Pufferparameter, wie pH-Wert oder Ionenzusammensetzung, kann erreicht werden, dass bei Anlegen von Spannung die Proteine in Richtung auf eine gewünscht Elektrode wandern. Insbesondere RNase-Enzyme sind dabei von Interesse.

Die Filtration wird solange durchgeführt, bis die RNA-Moleküle im Wesentlichen aus dem Gel eluiert sind. Die Gelelektrophorese bietet in diesem Zusammenhang den Vorteil, dass sie so effizient ist, dass nach nur einem Aufreinigungsschritt der Analyt in ausreichend reiner Form für eine direkte Hybridisierung vorliegt. Somit kann ein PCR-Schritt insbesondere im Zusammenhang mit natürlich amplifizierten RNA-Molekülen, wie der tmRNA, aufgrund der hohen Extraktionsund Aufreinigungsausbeute unterbleiben.

Insbesondere ist vorliegend ein Integriertes fluidisches Bauteil, wobei die Rezeptoreinrichtung betreibbar ist, ein Hybridisierungsexperiment durchzuführen.

Insbesondere ist auch vorliegend ein Integriertes fluidisches Bauteil wobei die elektrophoretische Gelfiltrationsstrecke (106) durch eine Sequenz von Gelen oder einen Gradienten von Gelen gebildet ist, die in ihrer Porengrösse, ihrer chemischen Zusammensetzung und/oder ihrem pH-Wert variieren und wobei einzelne Teile der Sequenz von Gelen durch Elektroden, die in der elektrophoretischen Gelfiltrationsstrecke integriert sind, unabhängig voneinander aktuierbar sind.

Weiterhin kann durch die spezifische Ausgestaltung der Gelfiltrationsstrecke eine zusätzliche Effizienzsteigerung erreicht werden. Beispielsweise kann ein Gelgradient oder eine Sequenz von verschiedenen Gelen vorgesehen sein. Die Sequenz von Gelen kann beispielsweise Gele mit absteigender Porengrösse beinhalten. Auch pH-Gradienten oder ionische Gradienten innerhalb eines Gels können verwendet werden.

Weiterhin ist es mit der erfindungsgemässen Lösung möglich, die Reinigung und die Detektion der aufgereinigten Analytmoleküle innerhalb ein und desselben mikrofluidischen integrierten Bauteils durchzuführen. Ein wesentlicher Vorteil der erfindungsgemässen Anordnung (...) besteht darin, dass die direkte Kopplung der Aufreinigung mit der Detektion ein reproduzierbares und automatisierbares Prinzip für die diagnostische Analyse bereitstellen kann.

Gemäß einer vorteilhaften Ausführungsform der vorliegenden Erfindung weist die Detektionseinrichtung eine Erkennungseinheit, beispielsweise eine Schicht, zum selektiven Anbinden der aufgereinigten Analytmoleküle auf. Derartige Erkennungsschichten, die auch als Rezeptorschichten bezeichnet werden, erlauben die selektive Detektion von Analytmolekülen und können mit heutiger Beschichtungstechnik in einem weiten Spektrum von Strukturformen aufgebracht werden. Insbesondere die Querempfindlichkeit kann mit einer derartigen Detektionseinrichtung (oder Rezeptoreinrichtung) erhöht werden, da möglicherweise in dem aufgereinigten Analytmaterial immer noch vorhandene Verunreinigungen nicht an die Erkennungsschicht binden.

Strukturiert man die Erkennungsschicht in Form eines Arrays und verwendet man unterschiedliche Schichten, die jeweils andere Analytmoleküle spezifisch binden, so kann mit Hilfe des erfindungsgemäßen Systems auch ein Multianalyterkennungsverfahren realisiert werden.

Gemäß einer ersten Ausführungsform der vorliegenden Erfindung können die selektiv gebundenen Analytmoleküle optisch mit Hilfe von Fluoreszenzmarkem, die beispielsweise dem Analyten beigegeben werden, erfasst werden. Dies stellt eine weitverbreitete und daher gut etablierte Methode zur Erfassung selektiv gebundener Analytmoleküle dar. Eine solche optische Auswertung kann durch Vorsehen einer entsprechenden Optik an dem mikrofluidischen Bauteil realisiert werden.

Gemäß einer weiteren vorteilhaften Ausführungsform der vorliegenden Erfindung ist vorgesehen, die selektiv gebundenen Analytmoleküle auf elektrischem Wege über Ladungseffekte zu detektieren. Dies hat den Vorteil, dass keine elektrooptischen Wandler und keine aufwändige Optik vorgesehen werden müssen und direkt ein elektrisches Ausgangssignal vorliegt. Eine solche elektrische Detektion kann sowohl auf dielektrischen Effekten wie auch auf Ladungen des Analyten basieren und es können potentiometrische oder impedanzanalysierende Verfahren eingesetzt werden. Zusammen mit entsprechenden elektrochemischen Mediatoren können auch amperometrische Detektionsverfahren Anwendung finden.

Ein Beispiel für eine elektrische Detektionseinrichtung, welche die selektiv gebundenen Analytmoleküle über Ladungseffekte detektiert, ist ein Biosensor auf der Basis synthetischer ligandengesteuerter Ionenkanäle. Derartige Biosensoren unter Verwendung ligandengesteuerter Ionenkanäle (synthetic ligand gated ion Channel, SLIC) basieren auf dem Prinzip, dass die Durchlässigkeit von Ionenkanälen in einer Lipidmembran durch das spezifische Anbinden von Analytmolekülen an entsprechende Rezeptoren gesteuert wird.

Damit kann die Affnitätsreaktion zwischen dem immobilisierten Rezeptor und dem sich anbindenden Analytmolekül als Änderung in der Impedanz des Gesamtsystems gemessen werden, wenn auf einer Elektrode eine entsprechend ausgestattete Lipidschicht immobilisiert wird. Die Lipiddoppelschicht arbeitet dabei sowohl als elektrische Isolation wie auch als Blockingreagens zum Verhindern unspezifischer Adsorption. Da ein Stromfluss nur durch die Ionenkanäle hindurcherfolgen kann, kann mit einem derartigen Sensor eine extrem empfindliche Detektion von Bindungsereignissen durchgeführt werden. Das Prinzip eines solchen Sensors ist beispielsweise in Samuel Terrettaz et al.: "Highly Electrically Insulating Tethered Lipid Bilayers for Probing the Function of Ion Channel Proteins", Langmuir 2003, 19, 5567-5569, gezeigt, wobei hier die Detektion von Proteinen durchgeführt wurde.

Alternativ können auch massensensitive Biosensoren, beispielsweise Quarzkristallmikrowaagen, Oberflächenweitenbauelemente oder Dünnschichtresonatoren, zur Detektion des aufgereinigten Analyten verwendet werden.

Ein weiteres mögliches Detektionsverfahren ist das Prinzip der Oberflächenplasmonenresonanz (Surface Plasmon Resonance, SPR). In einem SPR-System werden als integrierte Rezeptoreinheit Transducer in Form von Goldinseln vorgesehen, und eine externe Detektionseinheit misst die Änderungen in dem SPR-Winkel als Funktion der gebundenen Analytmoleküle.

Die vorliegende Erfindung lässt sich in besonders vorteilhafter Weise zur Aufreinigung und Detektion kleiner Nukleinsäuremoleküle, insbesondere Ribonukleinsäuremoleküle, einsetzen. Speziell zur genomischen Identifikation von Bakterien lässt sich tmRNA (transfer messenger ribonucleic acid), eine bakterielle RNA, die sowohl Botenwie auch Transfereigenschaften hat, verwenden. Insbesondere ist vorliegend ein Integriertes fluidisches Bauteil, wobei die Zellenmischung nachzuweisende Viren und/oder Bakterien enthält.

Insbesondere ist auch vorliegend ein Integriertes fluidisches Bauteil, wobei die Probe eine humane Körperflüssigkeit, vorzugsweise Blut oder Speichel, ist und die Ribonukleinsäuremoleküle Bestandteil nichthumaner Spezies, vorzugsweise Viren oder Bakterien, sind.

Insbesondere ist auch vorliegend ein Integriertes fluidisches Bauteil, wobei die Ribonukleinsäuremoleküle (104) Transfer-Messenger-RNA, tmRNA, umfassen.

TmRNA besteht typischerweise aus relativ wenigen Nukleotiden (< 400) und wurde in allen bislang sequenzierten Bakterienspezies identifiziert (siehe W. Schönhuber et al.: "Utilization of tmRNA sequences for bacterial Identification", BMC Microbiology, 2001, I-20). Da die tmRNA ein in natürlicher weise amplifiziertes Molekül ist (in E. coli sind beispielsweise 1000 Kopien pro Zelle vorhanden), ist sie ein interessanter Analyt für die genetische Identifikation von Bakterien. Mit der erfindungsgemässen mikrofluidischen Aufreinigung kann in vorteilhafter Weise auf eine Polymerase Chain Reaction, PCR, für den Nachweis verzichtet werden.

Bei Verwendung des erfindungsgemässen integrierten mikrofluidischen Bauteils zum Nachweis bakterieller tmRNA erfolgt die Detektion der aufgereinigten RNA über ein Hybridisierungsexperiment. Im Falle des SLIC-Biosensors ist das jeweils komplementäre Oligonukleotid, ein so genanntes "capture oligo", an den Ionenkanälen immobilisiert und das Hybridisierungsereignis steuert die Durchlässigkeit der Ionenkanäle für vorhandene Ionen und beeinflusst damit die Impedanz der Sensoranordnung.

Gemäss einer vorteilhaften Weiterbildung der vorliegenden Erfindung umfasst das integrierte mikrofluidische Bauteil zusätzliche Konditionierungseinrichtungen zum Behandeln der noch nicht aufgereinigten Probe und/oder zum Behandeln der aufgereinigten Analytmoleküle. So kann die Konditionierungseinrichtung beispielsweise eine Aufschlusseinrichtung zum Aufschliessen der Probe in der Probenkammer umfassen. Gemäss dieser Ausführungsform können unmittelbar die bakteriellen Zellen in die Probenkammer eingegeben werden und beispielsweise mittels einer ACthermoelektrischen Lysis aufgespalten werden.

Ein weiterer Aspekt betrifft ein Integriertes fluidisches Bauteil, wobei das Bauteil weiterhin mindestens eine integrierte Konditionierungsvorrichtung zum Behandeln der Probe und/oder zum Behandeln der aufgereinigten Ribonukleinsäuremoleküle umfasst und wobei die Konditionierungsvorrichtung eine Aufschlusseinrichtung zum Aufschliessen der Probe in der Probenkammer umfasst.

Ein Integriertes fluidisches Bauteil, wobei das Bauteil weiterhin mindestens eine integrierte Konditionierungsvorrichtung zum Behandeln der Probe und/oder zum Behandeln der aufgereinigten Ribonukleinsäuremoleküle umfasst, wobei die Konditionierungsvorrichtung eine Aufschlusseinrichtung zum Aufschliessen der Probe in der Probenkammer umfasst, und wobei die Aufschlusseinrichtung betreibbar ist, eine Zelllyse, vorzugsweise eine elektrische, thermische, mechanische, ultraschallgestützte, osmotische, enzymatische und/oder chemische Zelllyse, durchzuführen und wobei die Probenkammer mindestens eine Elektrode zum Durchführen der Zelllyse ausweist, ist beschrieben.

Ein Integriertes fluidisches Bauteil, wobei das Bauteil weiterhin mindestens eine integrierte Konditionienrngsvorrichtung zum Behandeln der Probe und/oder zum Behandeln der aufgereinigten Ribonukleinsäuremoleküle umfasst, wobei die eine Aufschlusseinrichtung zum Aufschliessen der Probe in der Probenkammer umfasst, wobei die Aufschlusseinrichtung betreibbar ist, eine Zelllyse, vorzugsweise eine elektrische, thermische, mechanische, ultraschallgestützte, osmotische, enzymatische und/oder chemische Zelllyse, durchzuführen, wobei die Probenkammer mindestens eine Elektrode gebildet ist zum Durchführen der Zelllyse ausweis und wobei die mindestens eine Elektrode durch coplanar struktuirete Elektrode gebildet ist, ist beschrieben.

Ein Integriertes fluidisches Bauteil, wobei das Bauteil weiterhin mindestens eine integrierte Konditionierungsvorrichtung zum Behandeln der Probe und/oder zum Behandeln der aufgereinigten Ribonukleinsäuremolektlle umfasst, wobei die

Konditionierungsvorrichtung eine Aufschlusseinrichtung zum Aufschliessen der Probe in der Probenkammer umfasst, wobei die Aufschlusseinrichtung betreibbar ist, eine Zelllyse, vorzugsweise eine elektrische, thermische, mechanische, ultraschallgestützte, osmotische, enzymatische und/oder chemische Zelllyse, durchzuführen, wobei die Probenkammer mindestens eine Elektrode der Zelllyse ausweis und wobei die mindestens eine Elektrode eine Vielzahl von Elektroden, die in unterschiedlichen Ebenen, vorzugsweise auf einer Oberseite und einer Unterseite eines Substrats, angeordnet sind, umfasst, ist beschrieben.

Ein weiterer Aspekt betrifft ein Integriertes fluidisches Bauteil, wobei das Bauteil weiterhin mindestens eine integrierte Konditionierungsvorrichtung zum Behandeln der Probe und/oder zum Behandeln der aufgereinigten Ribonukleinsäuremoleküle umfasst, wobei die thermische Zelllyse durch Anlegen eines Wechselstroms erfolgt.

Ein weiterer Aspekt betrifft ein Integriertes fluidisches Bauteil, wobei das Bauteil weiterhin mindestens eine integrierte Konditionierungsvorrichtung zum Behandeln der Probe und/oder zum Behandeln der aufgereinigten Ribonukleinsäuremoleküle umfasst, wobei die thermische Zelllyse durch Anlegen eines Wechselstroms erfolgt, und wobei die Probe eine elektrische Leitfähigkeit aufweist, die geringer ist als eine elektrische Leitfähigkeit der aufzuschliessenden Zellen. Die lysierte Zellmischung wird anschliessend erfindungsgemäss unmittelbar durch eine elektrophoretische Filtration aufgereinigt. Durch die direkt nach dem Zellaufschluss erfolgende Trennung bleibt eventuell dennoch vorhandener RNAse keine Zeit für eine schädliche Degradation. Überhaupt stellt der Zeitfaktor für die erfindungsgemässen Prinzipien einen wesentlichen Parameter dar: Je länger die Elutionszeit beispielsweise ist, desto höher ist die Ausbeute, desto geringer aber auch die Reinheit des Analytmaterials.

Für eine effiziente Kombination von Lyse und elektrophoretische Aufreinigung können verschiedene Protokolle verwendet werden. Es kann entweder als einfachste Variante unmittelbar an den Lyseschritt anschliessend die erfindungsgemässe elektrophoretische Aufreinigung erfolgen, oder aber zunächst eine Lyse der Zellen in einem elektrophoretischen Feld-gradienten erfolgen, so dass die beiden Prozesse parallel ablaufen. Zusätzlich können RNase-hemmende Enzyme und Chemikalien zugegeben werden, um den Prozess weiter zu optimieren.

In der Sammelkammer kann dann die aufgereinigte RNA auf einem Biosensorarray hybridisieren und das Hybridisierungsereignis als elektrisches oder optisches Ausgangssignal weiterverarbeitet werden. Selbstverständlich können aber auch alternative Konditionierungseinrichtungen vorgesehen sein, beispielsweise können Separations-, Filtrations-, Waschund Fokussierungsschritte, weitere Transportschritte, Aufkonzentration, Sedimentierung, Adsorption oder Degradierung durchgeführt werden. Diese zusätzlichen Schritte können entweder in der Probenkammer oder in der Sammelkammer erfolgen. Auf diese Weise kann das integrierte mikrofluidische Bauteil auch an komplexere biochemische Detektionsroutinen angepasst werden. Darüber hinaus ist einem Fachmann klar, dass auch Prinzipien der integrierten Realtime-PCR als Konditionierung vorgesehen werden können.

Ausserdem kann eine so genannte Realtime-NASBA (Nucelic Acid Sequence Base Amplification) alternativ zur Realtime-PCR vorgesehen sein, wobei die NASBA eine Amplifikationstechnik ist, die bei konstanter Temperatur stattfindet und spezifisch für RNA-Moleküle ist. Ebenso wie bei der PCR kann die NASBA unmittelbar durch Verwendung von fluoreszierenden Markermolekülen überwacht werden.

Ein weiterer Aspekt ist ein Verfahren zum Aufreinigen von Ribonukleinsäuremolekülen mittels eines integrierten fluidischen Bauteils, das die folgenden Schritte umfasst: Befüllen einer auf dem integrierten fluidischen Bauteil angeordneten Probenkammer mit einer die Ribonukleinsäuremoleküle enthaltenden Probe, wobei die Probe ein Zelllysat oder eine Zellenmischung ist; Transportieren der Probe entlang einer auf dem integrierten fluidischen Bauteil angeordneten elektrophoretischen Gelfiltrationsstrecke, wobei die elektrophoretische Gelfiltrationsstrecke unmittelbar mit der Probenkammer verbunden ist, so dass die Probe unmittelbar durch die elektrophoretische Filtration aufgereinigt wird, und wobei die Gelfiltrationsstrecke angepasst ist, die Ribonukleinsäuremoleküle von der Probe abzutrennen; Sammeln der aufgereinigten Ribonukleinsäuremoleküle in einer auf dem integrierten fluidischen Bauteil angeordneten Sammelkammer; Erfassen der Anwesenheit und/oder der Konzentration der aufgereinigten Ribonukleinsäuremoleküle mittels einer auf dem mikrofluidischen Bauteil integrierten Rezeptoreinrichtung, wobei die Rezeptoreinrichtung eine arrayförmig strukturierte Erkennungsschicht zum selektiven Anbinden der aufgereinigten Ribonukleinsäuremolekllle umfasst.

Insbesondere ist vorliegend ein Verfahren beschrieben wobei die Rezeptoreinrichtung eingerichtet ist, selektiv gebundene Ribonukleinsäuremoleküle optisch mittels Fluoreszenz oder elektrisch über einen Ladungseffekt, vorzugsweise impedanzspektroskopisch potentiometrisch oder amperometrisch zu detektieten.

Insbesondere ist vorliegend ein Verfahren beschrieben wobei die Rezeptoreinrichtung eingerichtet ist, selektiv gebundene Ribonukleinsäuremolekale optisch mittels Fluoreszenz oder elektrisch über einen Ladungseffekt, vorzugsweise impedanzspektroskopisch potentiometrisch oder amperometrisch zu detektieren, wobei die Rezeptoreinrichtung einen Biosensor auf der Basis synthetischer ligandengesteuerter Ionenkanäle umfasst.

Insbesondere ist vorliegend ein Verfahren beschrieben wobei die Rezeptoreinrichtung eingerichtet ist, selektiv gebundene Analytmoleküle mittels Oberflächenplasmonenresonanz zu detektieren Insbesondere ist vorliegend ein Verfahren der Erfindung, wobei die elektrophoretische Gelfiltrationsstrecke durch eine Sequenz von Gelen oder einen Gradienten von Gelen gebildet ist, die in ihrer Porengrösse, ihrer chemischen Zusammensetzung und/oder ihrem pH-Wert variieren und wobei einzelne Teile der Sequenz von Gelen durch Elektroden, die in der elektrophoretischen Gelfiltrationsstrecke integriert sind, unabhängig voneinander aktuierbar sind. Insbesondere ist vorliegend ein Verfahren der Erfindung, wobei das Gel über einen Seitenkanal mittels eines externen Dosiersystems, vorzugsweise einer Pipette, einbringbar ist.

Insbesondere ist vorliegend ein Verfahren beschriebe wobei das Gel über einen Seitenkanal mittels eines externen Dosiersystems, vorzugsweise einer Pipette, einbringbar ist und das Gel durch eine räumliche Steuerung infolge der Anwesenheit von Phasenführungen strukturierbar ist

Insbesondere ist vorliegend ein Verfahren der Erfindung, wobei die elektrophoretische Gelfiltrationsstrecke mindestens einen Seitenkanal aufweist, der durch mindestens eine zusätzlichen Elektrode aktuierbar ist.

Insbesondere ist vorliegend ein Verfahren beschriebe wobei die elektrophoretische Gelfiltrationsstrecke mindestens einen Seitenkanal aufweist, der durch mindestens eine zusätzliche Elektrode aktuierbar ist und der mindestens eine Seitenkanal so ausgebildet ist, dass er für eine Vorabtrennung von unerwüschten Probenbestandteilen, die eine höhere Migrationsgeschwindigkeit haben als die Ribonukleinsäuremoleküle, betreibbar ist. Insbesondere ist vorliegend ein Verfahren der Erfindung wobei die elektrophoretische Gelfiltrationsstrecke mindestens einen Seitenkanal aufweist, der durch mindestens eine zusätzliche Elektrode aktuierbar ist, und wobei der mindestens eine Seitenkanal betreibbar ist, um eine Sequenz von Gelen unabhängig voneinander zu aktuieren.

Insbesondere ist vorliegend ein Verfahren beschrieben wobei die Rezeptoreinrichtung betreibbar ist, ein Hybridisierungsexperiment durchzuführen.

Insbesondere ist vorliegend ein Verfahren beschrieben wobei die Probe eine humane Körperflüssigkeit, vorzugsweise Blut oder Speichel, ist und die Ribonukleinsäutemoleküle Bestandteil nichthumaner Spezies, vorzugsweise Viren oder Bakterien, sind.

Insbesondere ist vorliegend ein Verfahren der Erfindung wobei Ribcnukleinsäuremoleküle Transfer-Messenger-RNA, tmRNA, umfassen.

Insbesondere ist vorliegend ein Verfahren beschrieben wobei das Bauteil weiterhin mindestens eine integrierte Konditionierungsvorrichtung zum Behandeln der Probe und/oder zum Behandeln der aufgereinigten Ribonukleinsäutemolekule umfasst.

Insbesondere ist vorliegend ein beschrieben wobei das Bauteil weiterhin mindestens eine integrierte Konditionierungsvorrichtung zum Behandeln der Probe und/oder zum Behandeln der aufgereinigten Ribonukleinsäuremoleküle umfasst und die Konditionierungsvorrichtung eine Aufschlusseinrichtung zum Aufschliessen der Probe in der Probenkammer umfasst.

Insbesondere ist vorliegend ein Verfahren der Erfindung, wobei das Bauteil weiterhin mindestens eine integrierte Konditionierungsvorrichtung zum Behandeln der Probe und/oder zum Behandeln der aufgereinigten Ribonukleinsäuremoleknie umfasst, die Konditionierungsvorrichtung eine Aufschlusseinrichtung zum Aufschliessen der Probe in der Probenkammer umfasst, die Aufschlusseinrichtung betreibbar ist, eine Zelllyse, vorzugsweise eine elektrische, thermische, mechanische, ultraschallgestützte, osmotische, enzymatische und/oder chemische Zelllyse, durchzuführen.

Insbesondere ist vorliegend ein Verfahren beschrieben wobei das Bauteil weiterhin mindestens eine integrierte Konditionierungsvorrichtung zum Behandeln der Probe und/oder zum Behandeln der aufgereinigten Ribonukleinsäuremoleküle umfasst, die Konditionierungsvorrichtung eine Aufschlusseinrichtung zum Aufschliessen der Probe in der Probenkammer umfasst und die Aufschlusseinrichtung betreibbar ist, eine Zelllyse, vorzugsweise eine elektrische, thermische, mechanische, ultraschallgestützte, osmotische, enzymatische und/oder chemische Zelllyse, durchzuführen, und wobei die Probenkammer mindestens eine Elektrode zum Durchführen der Zelllyse aufweist.

Insbesondere ist vorliegend ein Verfahren beschrieben wobei das Bauteil weiterhin mindestens eine integrierte Konditionierungsvorrichtung zum Behandeln der Probe und/oder zum Behandeln der aufgereinigten Ribonukleinsäuremoleküle umfasst, die Konditionierungsvorrichtung eine Aufschlusseinrichtung zum Aufschliessen der Probe in der Probenkammer umfasst, die Aufschlusseinrichtung betreibbar ist, eine Zelllyse, vorzugsweise eine elektrische, thermische, mechanische, ultraschallgestützte, osmotische, enzymatische und/oder chemische Zelllyse, durchzuführen, die Probenkammer mindestens eine Elektrode zum Durchrühren der Zelllyse aufweist und wobei die mindestens eine Elektrode durch coplanar strukturierte Elektroden gebildet ist. Insbesondere ist vorliegend ein Verfahren beschrieben wobei das Bauteil weiterhin mindestens eine integrierte Konditionierungsvorrichtung zum Behandeln der Probe und/oder zum Behandeln der aufgereinigten Ribonukleinsäuremoleküle umfasst, die Konditionierungsvorrichtung eine Aufschlusseinrichtung zum Aufschliessen der Probe in der Probenkammer umfasst, die Aufschlusseinrichtung betreibbar ist, eine Zelllyse, vorzugsweise eine elektrische, thermische, mechanische, ultraschallgestützte, osmotische enzymatische und/oder chemische Zelllyse, durchzuführen, die Probenkammer mindestens eine Elektrode zum Durchführen der Zelllyse aufweist und wobein die mindestens eine Elektrode durch eine Vielzahl von Elektroden, die in unterschiedlichen Ebenen, vorzugsweise auf einer Oberseite und einer Unterseite eines Substrats, angeordnet sind, gebildet ist.

Insbesondere ist vorliegend ein Verfahren beschrieben wobei das Bauteil weiterhin mindestens eine integrierte Konditionierungsvorrichtung zum Behandeln der Probe und/oder zum Behandeln der aufgereinigten Ribonukleinsäuremolekale umfasst und die Konditionierungsvorrichtung eine Aufschlusseinrichtung zum Aufschliessen der Probe in der Probenkammer umfasst, die Aufschlusseinrichtung betreibbar ist, eine Zelllyse, vorzugsweise eine elektrische, thermische, mechanische, ultraschallgestützte, osmotische, enzymatische und/oder chemische Zelllyse, durchzuführen und wobei die thermische Zelllyse durch Anlegen eines Wechselstroms erfolgt.

Insbesondere ist vorliegend ein Verfahren der Erfindung, wobei das Bauteil weiterhin mindestens eine integrierte Konditionierungsvorrichtung zum Behandeln der Probe und/oder zum Behandeln der aufgereinigten Ribonukleinsäuremoleküle umfasst und die Konditionierungsvorrichtung eine Aufschlusseinrichtung zum Aufschliessen der Probe in der Probenkammer umfasst, die Aufschlusseinrichtung betreibbar ist, eine Zelllyse, vorzugsweise eine elektrische, thermische, mechanische, ultraschallgestützte, osmotische, enzymatische und/oder chemische Zelllyse, durchzuführen, die thermische Zelllyse durch Anlegen eines Wechselstroms erfolgt und wobei die Probe eine elektrische Leitfähigkeit aufweist, die geringer ist als eine elektrische Leitfähigkeit der aufzuschliessenden Zellen.

Insbesondere ist vorliegend ein Verfahren beschrieben wobei die Rezeptoreinrichtung durch ein von dem übrigen Bauteil getrenntes Modul gebildet ist, das vor einer Messung mit dem Bauteil verbindbar ist.

Insbesondere ist vorliegend ein Verfahren beschrieben wobei eine Bewegungsrichtung der Probe durch die Separationsstrecke quer zu einer Erkennungsschicht der Rezeptoreinheit verläuft.

Insbesondere ist vorliegend ein Verfahren der Erfindung wobei eine Bewegungsrichtung der Probe durch die Separationsstrecke quer zu einer Erkennungsschicht der Rezeptoreinheit verläuft und wobei die Separationsstrecke konisch geformt ist und die Probenkammer ein grösseres Volumen hat als die Sammelkammer.

Anhand der in den beiliegenden Zeichnungen dargestellten vorteilhaften Ausgestaltungen wird die vorliegende Erfindung im Folgenden näher erläutert. Ähnliche oder korrespondierende Einzelheiten des erfindungsgemässen Gegenstandes sind mit denselben Bezugszeichen versehen. Es zeigen:
- **Fig. 1**: eine schematische Darstellung einer ersten Ausführungsform des erfindungsgemässen integrierten mikrofluidischen Bauteils im Ausgangszustand;
- **Fig. 2**: die Anordnung der Fig. 1 während eines Aufreinigungsprozesses der Analytmoleküle;
- **Fig. 3**: die Anordnung aus Fig. 1 während eines Detektionsprozesses der aufgereinigten Analytmoleküle; Fig. 4 eine Anordnung gemäss einer zweiten Ausführungsform in schematischer Darstellung nach dem Einfüllen der Probe;
- **Fig 4**: eine Anordnung gemäß einer zweiten Ausführungsform in schematischer Darstellung nach dem Einfüllen der Probe;
- **Fig. 5**: die Anordnung aus Fig. 4 während des Aufreinigungsprozesses;
- **Fig. 6**: die Anordnung aus Fig. 4 während des Detektionsprozesses;
- **Fig. 7**: eine schematische Darstellung einer Rezeptoreinheit zum selektiven Anbinden der aufgereinigten Analytmoleküle basierend auf synthetischen ligandengesteuerten Ionenkanälen vor dem Hybridisierungsprozess;
- **Fig. 8**: die Anordnung der Fig. 7 während des Anbindens der aufgereinigten RNAMoleküle;
- **Fig. 9**: die Anordnung der Fig. 7 nach dem Anbinden der aufgereinigten RNA-Moleküle;
- **Fig. 10**: eine schematische Darstellung einer weiteren vorteilhaften Ausführungsform des erfindungsgemässen integrierten mikrofluidischen Bauteils;
- **Fig.11**: die Anordnung der Fig. 10 nach dem Aufschliessen der Probe;
- **Fig. 12**: die Anordnung der Fig. 10 während der elektrophoretischen Auftrennung;
- **Fig. 13**: die Anordnung der Fig. 10 während des Detektionsvorgangs;
- **Fig. 14**: ein Flussdiagramm des Aufreinigungsund Detektionsvorgangs;
- **Fig. 15**: eine schematische Darstellung einer weiteren vorteilhaften Ausführungsform des erfindungsgemässen integrierten mikrofluidischen Bauteils, bei der die Separationsstrecke mehrere Gele mit unterschiedlicher Porengrösse aufweist;
- **Fig. 16**: die Anordnung der Figur 15 während des Aufreinigungsprozesses;
- **Fig.17**: die Anordnung aus Figur 15 nach abgeschlossenem Aufreinigungsprozess;
- **Fig.18**: eine schematische Darstellung einer weiteren vorteilhaften Ausführungsform des erfindungsgemäßen integrierten mikrofluidischen Bauteils mit mehrfachen Gelfiltrationsstrecken;
- **Fig. 19**: die Anordnung aus Figur 18 bei Anlegen einer Spannung an einer ersten Gelfiltratonsstrecke;
- **Fig. 20**: die Anordnung aus Fig. 18 bei Anlegen einer Spannung an einer zweiten Gelflitrationsstrecke;
- **Fig. 21**: eine schematische Darstellung einer weiteren vorteilhaften Ausführungsform, die einen Seitenkanal zur Vorabtrennung von schnelleren Spezies aufweist;
- **Fig. 22**: die Anordnung aus Figur 21 bei Anlegen einer Spannung zwischen der Probenkammer und einer ersten Sammelkammer;
- **Fig. 23**: die Anordnung aus Figur 21 bei Anlegen einer Spannung zwischen der Probenkammer und einer zweiten Sammelkammer;
- **Fig. 24**: eine schematische Darstellung einer verfeinerten Anordnung gemäß Figur 21 mit einer zusätzlichen Abfallkammer;
- **Fig. 25**: die Anordnung aus Figur 24 während eines ersten Auftrennungsschritts;
- **Fig. 26**: die Anordnung aus Figur 24 während eines zweiten Auftrennungsschritts;
- **Fig. 27**: ein elektrisches Ersatzschaltbild für die Probenkammer während der elektrischen Zelllyse;
- **Fig. 28**: eine schematische Darstellung des Strompfades bei Anlegen einer Gleichspannung;
- **Fig. 29**: eine schematische Darstellung des Strompfades für Wechselspannungen, wenn der Puffer leitfähiger ist als das Zellzytoplasma oder wenn zu niedrige Frequenzen gewählt werden;
- **Fig. 30**: eine schematische Darstellung des Strompfads für Wechselspannungen, wenn das Medium eine niedrigere Leitfähigkeit hat als das Zellzytoplasma;
- **Fig. 31**: eine schematische Darstellung einer ersten Elektrodenkonfiguration in einer geschlossenen Mikrokammer;
- **Fig. 32**: eine zweite Elektrodenkonfiguration für eine geschlossene Mikrokammer;
- **Fig. 33**: eine dritte Elektrodenkonfiguration für eine geschlossene Mikrokammer;
- **Fig. 34**: eine weitere Elektrodengeometrie für eine geschlossene Mikrokammer;
- **Fig. 35**: eine Draufsicht auf eine offene integrierte Elektrodenanordnung;
- **Fig. 36**: eine Schnittdarstellung der Anordnung aus Figur 35;
- **Fig. 37**: die Anordnung aus Figur 36 im befüllten Zustand;
- **Fig. 38**: eine schematische Darstellung einer weiteren vorteilhaften Ausführungsform des erfindungsgemäßen integrierten mikrofluidischen Bauteils;
- **Fig. 39**: eine schematische Darstellung einer weiteren vorteilhaften Ausführungsform;
- **Fig. 40**: eine schematische Darstellung einer weiteren vorteilhaften Ausführungsform;
- **Fig. 41**: einen Schnitt durch eine dreidimensionale Ausführungsform des erfindungsgemäßen mikrofluidischen Bauteils während der Montage;

- **Fig. 42**: die Anordnung aus Figur 41 im montierten Zustand während eines Zelllysevorgangs;
- **Fig. 43**: die Anordnung der Figur 42 zu Beginn eines Elektrophoresevorgangs;
- **Fig. 44**: die Anordnung aus Figur 42 gegen Ende des elektrophoretischen Auftrennungsvorgangs;
- **Fig. 45**: eine schematische Darstellung des Herstellungsvorgangs einer Gelstruktur zu einem ersten Zeitpunkt;
- **Fig. 46**: eine schematische Darstellung des Herstellungsvorgangs einer Gelstruktur zu einem späteren Zeitpunkt;
- **Fig. 47**: die fertig gestellte Gelstruktur der Figuren 45 und 46.

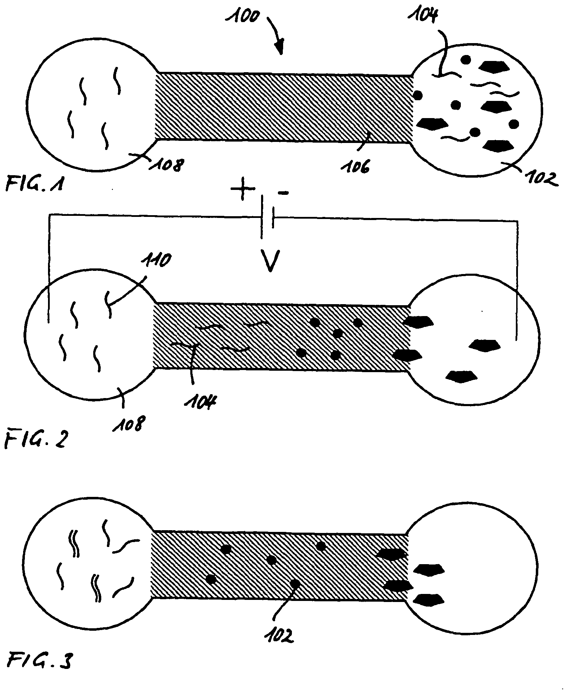

Der Aufbau und die Wirkungsweise des erfindungsgemäßen integrierten mikrofluidischen Bauteils 100 soll im Folgenden mit Bezug auf die Figuren 1 bis 3 näher erläutert werden.

Das mikrofluidische Bauteil 100 umfasst in seiner einfachsten Ausführungsform eine Probenkammer 102 zum Aufnehmen einer Probe, welche die Analytmoleküle 104 enthält. Dabei handelt es sich bei den Probenmolekülen 104 beispielsweise um RNA-Moleküle, wie z. B. tmRNA, die einen Nachweis von Bakterien ermöglichen. Das mikrofluidische Bauteil weist weiterhin eine Separationsstrecke 106 auf, entlang welcher die Probe bewegt und dadurch der Analyt 104 von den übrigen Probenbestandteilen getrennt wird. Die Probe kann eine Nukleinsäurenmischung, ein Zelllysat oder aber auch eine Zellmischung sein.

Die Separationsstrecke 106 ist gemäß der vorliegende Erfindung durch einen Gelfiltrationspfad, der eine oder mehrere Elektrophorese-Gelmatrizen aufweist, gebildet. Nach dem Anlegen der für die elektrophoretische Auftrennung erforderlichen Spannung zwischen Anfang und Ende der Separationsstrecke 106 bewegen sich die aufzureinigenden Analytmoleküle in Richtung auf eine Sammelkammer 108. Zur Detektion der aufgereinigten RNA-Moleküle ist in der Sammelkammer als Erkennungsschicht das komplementäre Oligonukleotid 110 immobilisiert. Dabei wird die Elektrophoresespannung solange aufrecht erhalten, bis eine ausreichende Menge RNA die Sammelkammer 108 erreicht hat und dann abgeschaltet, so dass die übrigen größeren Probenbestandteile 112 nicht bis in die Sammelkammer 108 gelangen. Unspezifische RNA-Stücke können an die Rezeptorschicht 110 nicht anbinden. Die erfolgte Hybridisierung wird anschließend mittels einer Fluoreszenzmarkierung optisch oder aber, wie dies im Folgenden noch genauer dargestellt wird, elektrisch mittels eines Biosensors auf der Basis ligandengesteuerter Ionenkanäle nachgewiesen.

Das erfindungsgemäße integrierte mikrofluidische Bauteil kann dabei ein planarer Mikrochip sein, wie er beispielsweise durch Strukturieren eines Pyrexglaswafers erzeugt werden kann. Dabei können die Probenkammer und die Sammelkammer integrierte Phasenführungsvorrichtungen für eine gesteuerte Flüssigkeitsein- und -ausgabe gemäß P. Vulto et al.: "Selective sample recovery of DEP-separated cells and particles by phaseguide-controlled laminar flow", J. Micromech. Microeng. 16, 2006, 1847-1853, enthalten.

Alternativ kann aber das integrierte mikrofluidische Bauteil auch eine dreidimensionale Ausgestaltung besitzen. Eine vertikale dreidimensionale Konstruktion eines integrierten Analysensystems bietet dabei im Vergleich zu einer planaren Anordnung etliche wesentliche Vorteile: Es kann ein größeres Oberfläche-zu-Volumen-Verhältnis erzeugt werden. Dies ist vorteilhaft, um die Kontaktoberfläche zwischen dem Gel und der Probenlösung zu erhöhen, da größere Oberflächen die Gefahr von Verklumpungen verringern. Weiterhin kann eine größere Menge anfänglicher Probenflüssigkeit eingesetzt werden, die durch die elektrophoretische Aufreinigung zu einem Mikrolitervolumen aufkonzentriert wird. Gele und Schichten aus einer Vielzahl von Gelen können bei einer 3-D-Anordnung auf einfache Weise durch Gießen hergestellt werden. Ein vertikales Setup ermöglicht außerdem ein modulares Konzept, bei dem die billigen und einfachen Probenvorkonditionierungselemente separat von den empfindlichen Biosensor- oder Affinitätssensorelementen hergestellt werden. Die beiden Teile werden kurz bevor eine Messung durchgeführt wird miteinander verbunden.

Schließlich kann bei einer dreidimensionalen Anordnung als Motor für die fluidische Aktuierung oder Sedimentation die Schwerkraft genutzt werden.

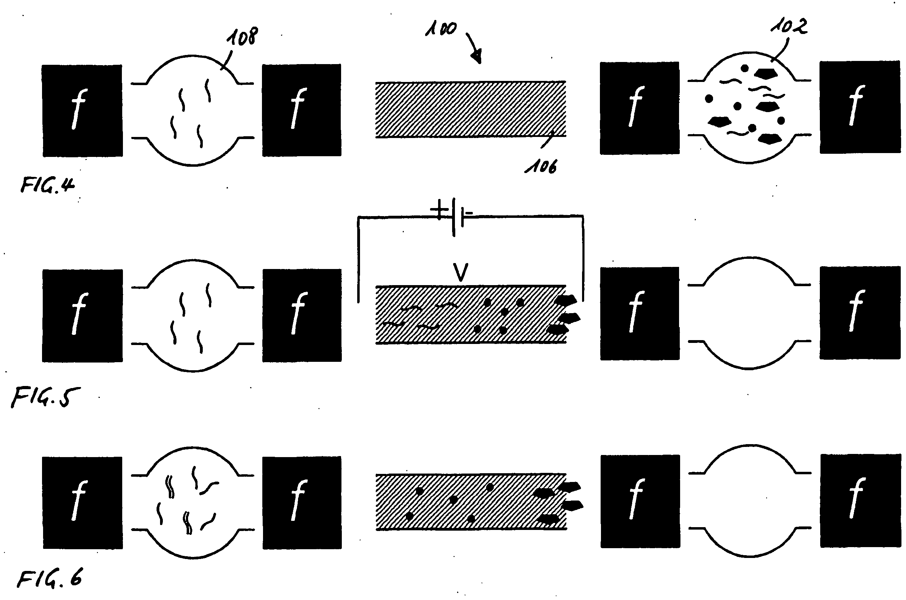

Die bisher beschriebene einfachste Anordnung zur integrierten Aufreinigung und Hybridisierung von tmRNA kann, wie in den Figuren 4 bis 6 gezeigt, um verschiedene zusätzliche Schritte erweitert werden. Die mit dem Buchstaben "f" bezeichnete Komponente symbolisiert dabei einen oder mehrere Konditionierungsschritte, wie beispielsweise eine Lyse, Separation, Filtration, einen Waschschritt, eine Fokussierung, einen Transportschritt, ein Aufkonzentrieren, eine Sedimentierung, eine Adsorption oder einen Degradationsschritt. Solche zusätzlichen Schritte können sowohl innerhalb der Probenkammer 102, wie auch innerhalb der Sammelkammer 108 durchgeführt werden.

Beispielsweise kann in die Probenkammer eine Zellprobe eingefüllt werden. In einem anschließenden Konditionierungsschritt werden auf elektrischem, chemischem, thermischem, osmotischem oder mechanischem Wege die Zellen lysiert, um die RNA freizusetzen. Im nächsten Schritt wird die freigesetzte RNA entlang der Elektrophoresestrecke, wie in den bisherigen Figuren gezeigt, aufgereinigt. In der Sammelkammer 108 hybridisiert die RNA mit den immobilisierten Rezeptormolekülen, die beispielsweise in Arrayform auf einem Substrat aufgebracht sind. Die Detektion der hybridisierten Moleküle erfolgt entweder über eine Fluoreszenzmarkierung oder auf elektrischem oder massensensitivem Wege.

Dadurch, dass die Lyse und Aufreinigung in ein und demselben integrierten mikrofluidischen System stattfinden, kann die Zeit, in der die empfindliche RNA zersetzenden RNase-Molekülen ausgesetzt ist, auf ein Minimum reduziert werden und somit eine RNA-Degradation verhindert werden.

Eine noch komplexere Vorgehensweise könnte beinhalten, dass die Analytzellen zunächst mittels einer Dielektrophorese von anderen Zellen und dem Zellüberstand befreit werden und erst dann eine elektrische oder chemische Zelllyse erfolgt. Danach können große Proteine z. B. mittels einer selektiven Adsorption und einer nachfolgenden Sedimentation aus dem Probengemisch entfernt werden, bevor der eigentliche Elektrophoreseschritt entlang des Gelpfades durchgeführt wird. Nach dem Sammeln in der Sammelkammer 108 kann eine Realtime-PCR durchgeführt werden, indem die Probe auf verschiedene PCR-Wells verteilt wird und eine Affinitätsreaktion, Amplifikation und Fluoreszenzdetektion durchgeführt wird.

Grundsätzlich ist außerdem einem Fachmann klar, dass selbstverständlich nicht nur eine einzige Separationsstrecke, sondern deren mehrere vorgesehen sein können, die beispielsweise in eine Vielzahl von Sammelkammern münden und die Untersuchung auf mehrere Analyten innerhalb derselben Probe ermöglichen. Alternativ oder zusätzlich kann auch eine Vielzahl von Probenkammern vorgesehen sein, um verschiedene Proben simultan analysieren zu können. Weiterhin können als Analytmoleküle jede Art von kleiner RNA, DNA, Peptide oder Proteine in Frage kommen.

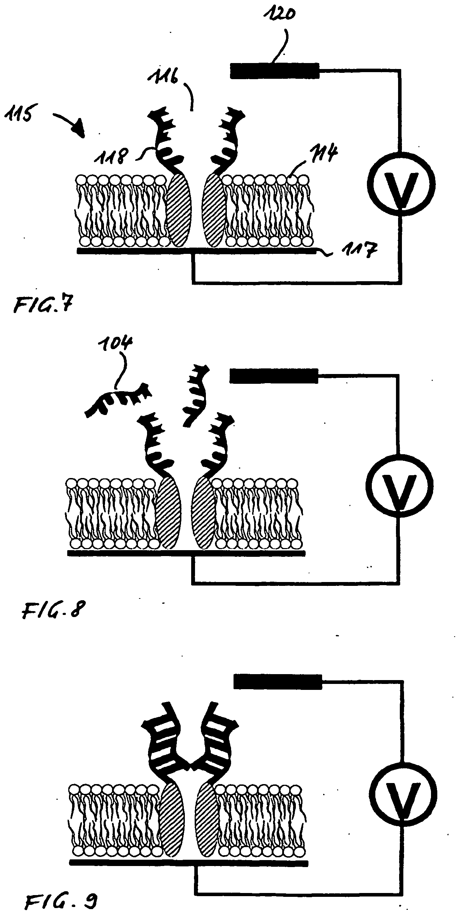

Mit Bezug auf die Figuren 7 bis 9 soll im Folgenden eine mögliche Ausführungsform eines Biosensors zur Detektion von tmRNA-Molekülen in der Sammelkammer erläutert werden.

Dabei basiert der verwendete Biosensor 115 auf dem Prinzip des synthetischen ligandengesteuerten Ionenkanals (synthetic ligand gated ion channel, SLIC). Ein derartiger SLIC-Biosensor 115 umfasst eine Lipiddoppelschicht 114, in der die Ionenkanäle 116 als die Durchlässigkeit steuernde Rezeptoren Fangoligonukleotide 118 tragen. Als Messsignal wird beispielsweise die Impedanz zwischen dem Substrat 117 und einer Gegenelektrode 120 ausgewertet. Wenn die zu detektierende tmRNA 104 selektiv an die komplementären Oligonukleotide bindet, ändert sich die Ionendurchlässigkeit der Kanäle 116 und damit auch die messbare Impedanz.

Da ein elektrischer Strom nur durch die Ionenkanäle 116 fließt, kann mit der erfindungsgemäßen Anordnung eine außergewöhnlich präzise Erfassung der Hybridisierungsereignisse erreicht werden.

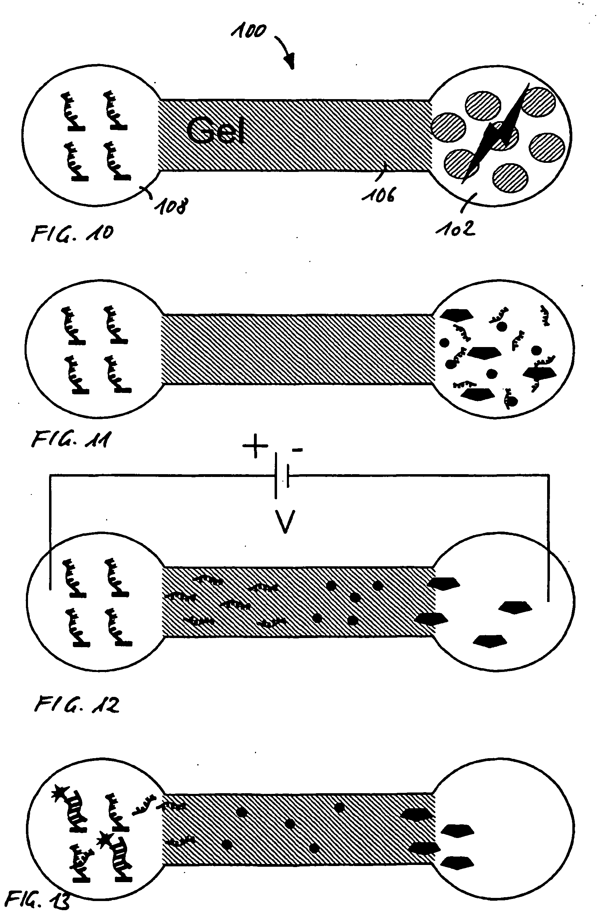

Eine mögliche Ausführungsform des integrierten mikrofluidischen Bauteils 100 unter Verwendung eines Affinitätsarrays, beispielsweise eines SLIC-Biosensors, ist in den Figuren 10 bis 13 skizziert. Dabei wird in der Probenkammer 102 zunächst eine Zelllyse von zu analysierenden ganzen Zellen 122 durchgeführt. Anschließend werden die zu detektierenden Analytmoleküle, beispielsweise RNA, insbesondere tmRNA-Bestandteile 104 entlang der Separationsstrecke 106 von den übrigen Zellbestandteileh getrennt und hybridisieren mit immobilisierten Rezeptormolekülen in der Probenkammer. Das Bindungsereignis führt zu einer Änderung der Impedanz des SLIC-Sensors und kann als elektrisches Ausgangssignal gemessen werden.

Die elektrophoretische Separationsstrecke 106 muss aber nicht unbedingt aus nur einem homogenen und symmetrischen Gel bestehen. Es können auch mehrfache Gele verwendet werden, die beispielsweise in der Porengröße, im Gelmaterial oder in der Pufferzusammensetzung variieren. Die Gele können in verschiedenen Formen und in verschiedenen Elektrodenkonfigurationen, die für die Aktuation verwendet werden, vorgesehen sein.

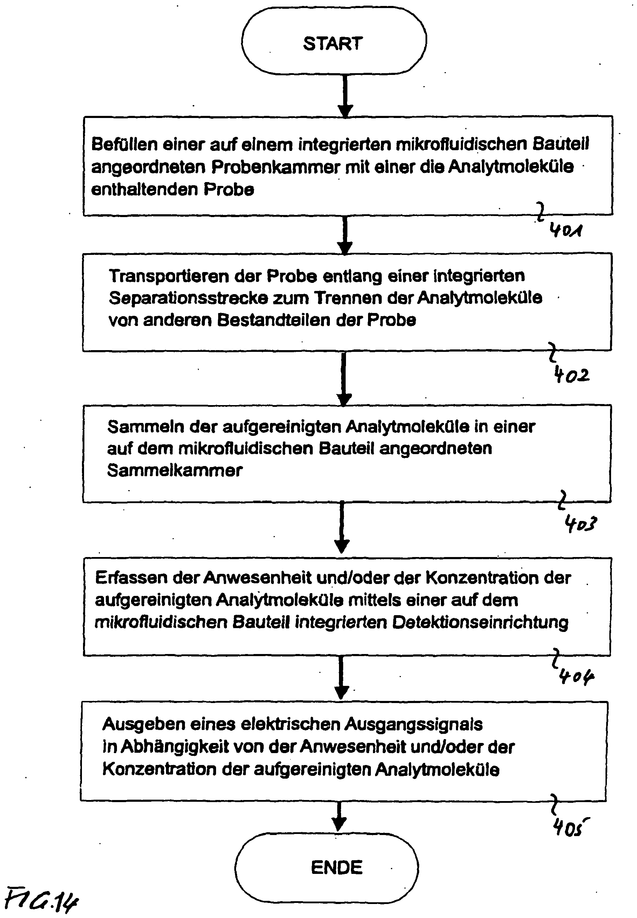

Die Funktionsweise der in den Figuren 10 bis 13 gezeigten Anordnung ist im Überblick in Fig. 14 als Flussdiagramm zusammengefasst. Das Nachweisverfahren beginnt mit dem Befüllen einer auf dem integrierten mikrofluidischen Bauteil angeordneten Probenkammer mit einer die Analytmoleküle enthaltenden Probe (Schritt 401). Durch Anlegen einer entsprechenden Spannung entlang der integrierten Separationsstrecke wird die Probe zum Trennen der Analytmoleküle von anderen Bestandteilen der Probe in Schritt 402 transportiert. Die aufgereinigten Analytmoleküle sammeln sich in einer ebenfalls auf dem mikrofluidischen Bauteil angeordneten Sammelkammer (Schritt 403). Anschließend kann die Anwesenheit und/oder die Konzentration der aufgereinigten Analytmoleküle mittels einer ebenfalls auf dem mikrofluidischen Bauteil integrierten Detektionseinrichtung erfasst werden (Schritt 404). In Schritt 405 schließlich wird ein elektrisches Ausgangssignal, das von der Anwesenheit und/oder der Konzentration der aufgereinigten Analytmoleküle abhängt, ausgegeben, wobei dieses Signal einer weiteren Signalverarbeitung zugänglich ist.

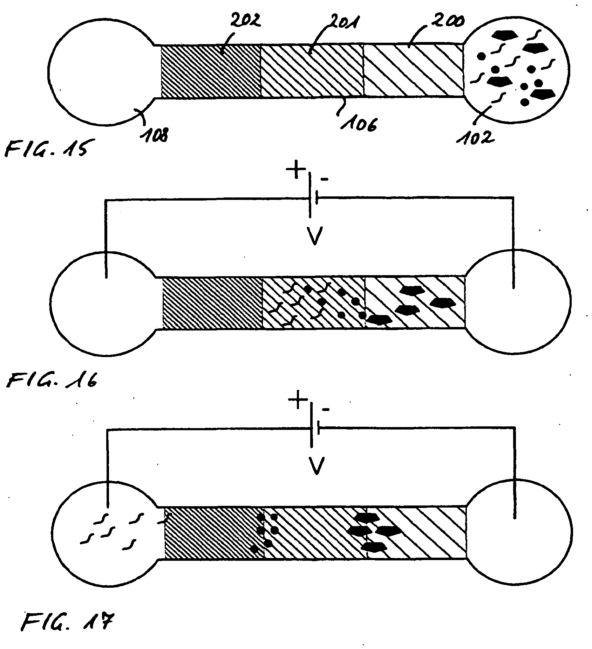

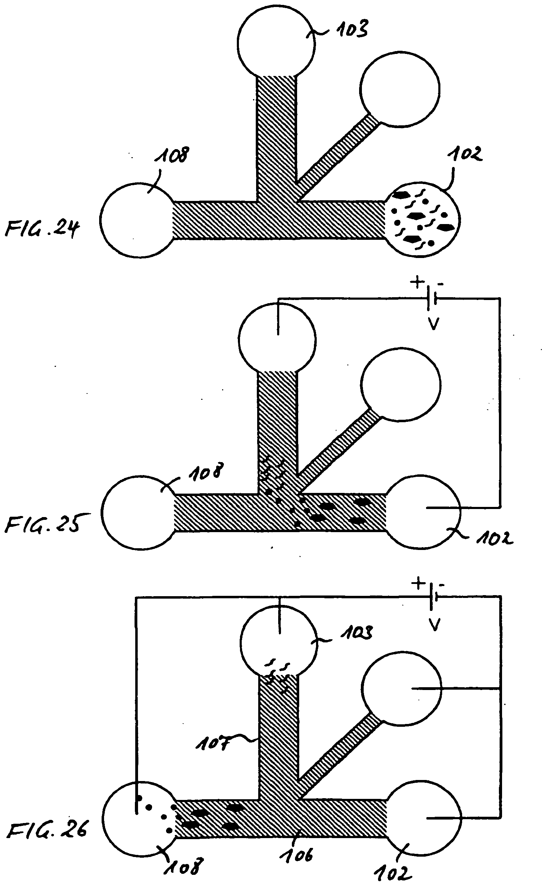

Mit Bezug auf die Figuren 15 bis 17 soll ein erstes Beispiel beschrieben werden, bei dem die Separationsstrecke 106 durch einen Gradienten von Gelen, die in der Dichte variieren, gebildet ist. Wie aus dieser Darstellung ersichtlich, ist die Separationsstrecke 106 aus drei verschiedenen Gelen zusammengesetzt, wobei für einen Fachmann klar ist, dass die Anzahl nur beispielhaft gewählt ist. Das erste Gel 200 hat eine große Porengröße, so dass in einem ersten Schritt große Verunreinigungen aus der Probe abgetrennt werden. Die nachfolgenden Gele 201, 202 haben jeweils eine abnehmende Porengröße und ihr Siebeffekt nimmt zu. Mechanismen, wie sie in den Figuren 24 bis 26 gezeigt sind, können beispielsweise eine Überladung des Gels verhindern oder eine Aufkonzentrierung des Probenmaterials vor der Separation bewirken.

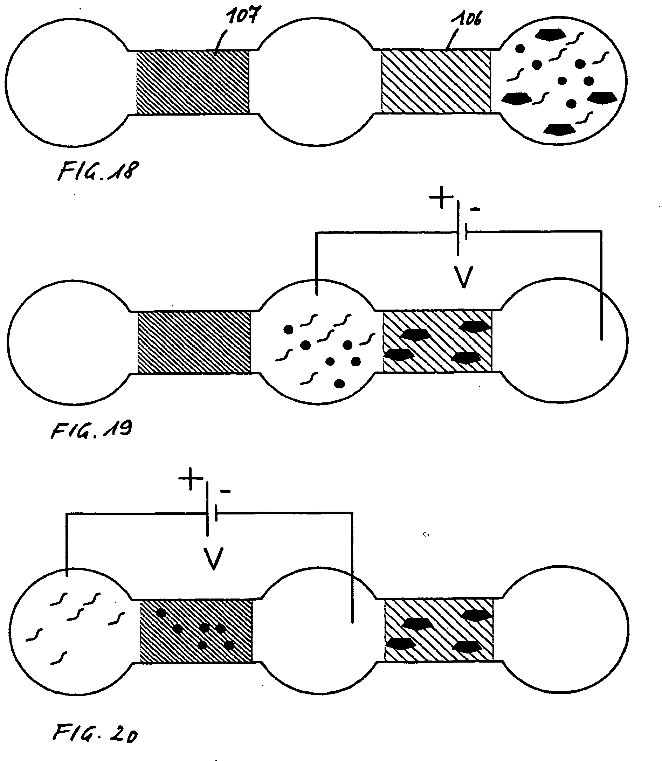

Ein weiteres Beispiel für multiple Gelseparationsstrecken ist in den Figuren 18 bis 20 gezeigt. Hier sind mehrere Gelstrecken 106, 107 und aufeinander folgende Sammelkammern hintereinander geschaltet. Selbstverständlich kann die Anzahl der seriell aufeinander folgenden Schritte beliebig den Anforderungen angepasst werden. In der gezeigten Ausführungsform sind zwei Gelseparationsstrecken als eine Kaskade geschaltet. Nach jedem Separationsschritt wird die Probe in einer Sammelkammer gesammelt. Die hier gezeigte Anordnung hat den Vorteil, dass sie eine höhere Reinheit des letztendlich gewonnen Analyten sowie eine größere Menge an Analytmaterial liefert. Die Vielzahl von Separationsstrecken 106, 107 können unabhängig voneinander, wie in den Figuren 18 bis 20 gezeigt, oder aber auch durch die gleichen Elektroden aktuiert werden. Weiterhin können die Gelseparationsstrecken 106, 107 identische Eigenschaften oder aber unterschiedliche Eigenschaften bezüglich Porengröße, Material oder Pufferzusammensetzung aufweisen.

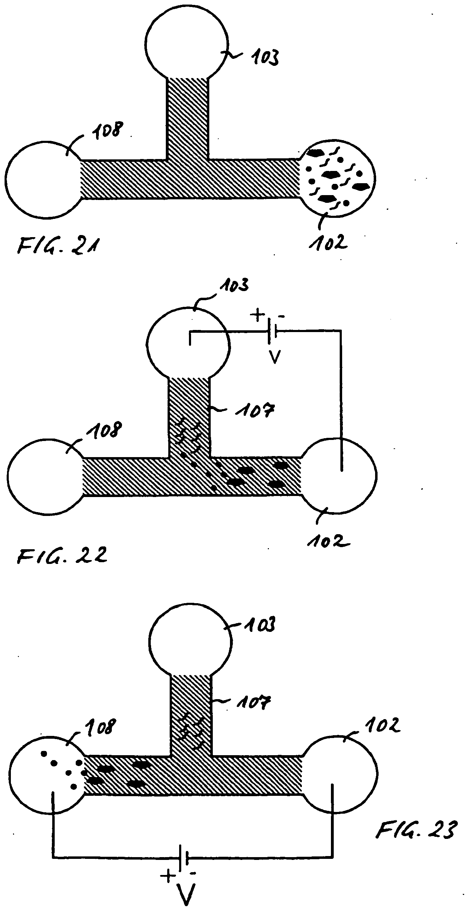

Bisher wurde stets davon ausgegangen, dass die Analytmoleküle die schnellsten Migrationseigenschaften im Vergleich zu den übrigen Probenbestandteilen aufweisen. Dies muss aber nicht unbedingt immer der Fall sein. Es ist durchaus möglich, dass verschiedene Probenbestandteile auch schnellere elektrophoretische Migrationseigenschaften haben als das aufzureinigende Analytmaterial. Dieses ebenfalls unerwünschte Material kann in vorteilhafter Weise durch Ablenken der Probe in einen Seitenkanal entfernt werden. Diese Vorgehensweise ist in den Figuren 21 bis 23 sowie 24 bis 26 skizziert.

Dabei wird, wie in den Figuren 21 bis 23 dargestellt, das elektrophoretische Potential zunächst zwischen der Probenkammer 102 und einem Seitenkanal 107 angelegt, bis die schnellwandernden Bestandteile sämtlich in den Seitenkanal 107 eingetreten sind. Dann wird, wie in Figur 23 gezeigt, das Potential zwischen der Sammelkammer 108 und der Probenkammer angelegt, bis das Analytmaterial in der Sammelkammer 108 in aufgereinigter Form vorliegt.

Die Figuren 24 bis 26 zeigen ein komplexeres Layout, bei dem ein zweites Potential zwischen einem zusätzlichen Kanal und der Probenkammer auf einer Seite und der Sammelkammer und dem ersten Kanal auf der anderen Seite angelegt wird. Dadurch kann verhindert werden, dass das voraufgetrennte Produkt wieder zurück in Richtung auf die Sammelkammer wandert. Stattdessen wird es in Richtung auf die Abfallkammer 103 wandern.

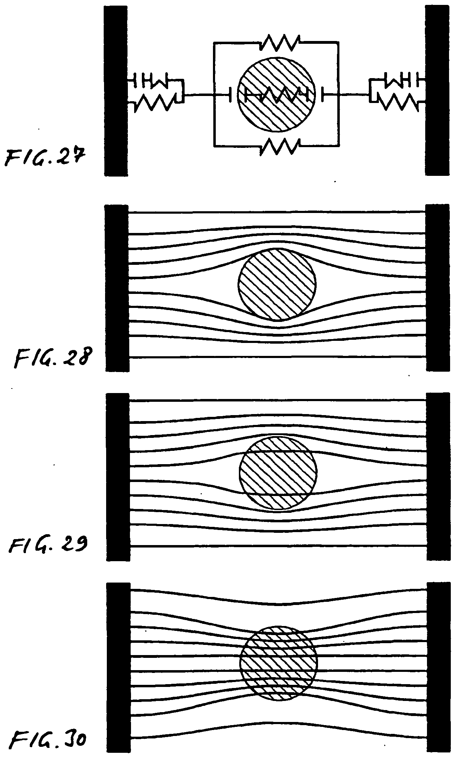

Um eine effiziente Zelllyse zu ermöglichen, kann ein Wechselstrom verwendet werden, der die Probe aufheizt. Schematisch ist dieser Prozess in den Figuren 27 bis 30 dargestellt. Dabei bezeichnet das Bezugszeichen 122 die aufzuschließende Zelle. Wenn der Puffer, in dem die Zellen 122 gelöst sind, eine niedrigere Leitfähigkeit als das Zytoplasma der Zellen aufweist, dann wird die Stromflussdichte innerhalb der Zellen am höchsten sein, wie dies in Figur 30 gezeigt ist. Das bedeutet, dass die volumenabhängige Wärmeerzeugung innerhalb der Zellen am höchsten ist, so dass die Zellen kalorisch aufgeschlossen werden, ohne die Umgebung um die Zellen zu sehr aufzuheizen. Dieses Verfahren ist besonders effektiv bei Zellen mit dicken Zellmembranen wie beispielsweise grampositive Bakterien. Zusätzlich können der Probenlösung auch verschiedene Lysereagenzien beigefügt werden. Diese elektrothermische Lyse in einer miniaturisierten Anordnung ist beispielsweise aus J. West et al.: "Accessing DNA by low voltage altemating current Joule effect heating", Analytica Chimica Acta 527 (2004) 1-12, bekannt. Im Unterschied zu der vorliegenden Erfindung werden dort jedoch nicht die Pufferbedingungen an die jeweiligen Zellleitfähigkeiten angepasst, um eine hohe Stromdichte innerhalb der Zellen zu erreichen. Weiterhin sind bei dieser bekannten Lösung keine Reinigungsschritte gemäß der vorliegenden Erfindung vorgesehen.

Der Ansatz einer lokalen Zellerwärmung mit einer niedrigeren Pufferleitfähigkeit im Vergleich zu der Zytoplasmaleitfähigkeit ist insbesondere in einem Mikrosystem vorteilhaft, da so eine Lyse durch Erhitzen realisiert werden kann, ohne die Probenflüssigkeit zum Kochen zu bringen, so dass eine Gasblasenbildung verhindert werden kann. Darüber hinaus kann die Probenlösung zum Kühlen der Lyseprodukte unmittelbar nach dem Lyseschritt verwendet werden, so dass eine schnelle RNA-Degradation verhindert werden kann. Die Kombination der lokalen Zellerwärmung mit einer zusätzlichen Probenkühlung, z. B. durch Peltier-Elemente erlaubt eine optimierte Kombination von thermischer Zelllyse und Pufferkühlung.

Gemäß einer weiteren vorteilhaften Ausführungsform kann die Probe außerdem in einem Puffer aufgelöst werden, der gleichzeitig eine Separationsmatrix darstellt. Beispielsweise können flüssige Polymerlösungen, wie sie für die Kapillarelektrophorese eingesetzt werden, auch in der vorliegenden integrierten Anordnung vorgesehen sein. Solche flüssigen Separationsmatrizen können verwendet werden, um direkt eine erste Auftrennung von sehr großen und kleineren Probenbestandteilen durchzuführen, um das Gel nicht zu überladen oder den Strompfad nicht durch eine Ansammlung von zu viel Material an einer Stelle zu blockieren. Gleichzeitig können solche Polymerlösungen auch als Zusatz zu dem Lysepuffer mit niedriger Leitfähigkeit verwendet werden.

Wie allgemein bekannt ist, ist die Elektrophorese ein Effekt, der von der Stärke des elektrischen Feldes abhängt. Diese Feldstärke wird unter anderem von dem angelegten Potential (bzw. dem Strom) und der Geometrie der Mikrostruktur, durch welche dieser Strom fließt, beeinflusst. Der elektrische Widerstand eines Mikrokanals hängt linear von dem Verhältnis zwischen seiner Länge und der Querschnittsfläche ab. Eine lokal erhöhte Querschnittsfläche, d.h. eine erhöhte Kanalbreite oder Höhe, führt zu einer niedrigeren lokalen Feldstärke. Da die elektrophoretische Mobilität der einzelnen Spezies nicht linear von der elektrophoretischen Feldstärke abhängt, wie dies beispielsweise aus J. Viovy: "Electrophoresis of DNA and other polyelectrolytes: physical mechanisms", Rev. Modem Physics, vol. 72, No. 3, 2000, 813-872, bekannt ist, kann ein solches Prinzip verwendet werden, um dadurch die elektrophoretische Auftrennung zu manipulieren. Ein Aufweiten der Sammelkammern nach der Gelaufreinigung kann beispielsweise verwendet werden, um die Migrationsgeschwindigkeit der Spezies zu verlangsamen und so zu verhindern, dass sie die Aktuationselektrode tatsächlich erreichen.

Die Aktuationselektroden für die elektrophoretische Auftrennung können entweder innerhalb der Mikrostruktur integriert sein oder aber als externe Elektroden ausgebildet sein. Der Vorteil integrierter Elektroden in einer geschlossenen Kammer besteht darin, dass das Probenvolumen durch die Kammer und Elektrodengeometrie exakt definiert ist. Integrierte Elektroden in einem geschlossenen Mikrokanal verhindern außerdem ein Verdunsten der Probenflüssigkeit. Ein weiterer Vorteil integrierter Elektroden ist, dass sie nach dem Benützen entsorgt werden und nicht wieder verwendet werden müssen.

Ein Nachteil von Elektroden in einem geschlossenen Raum ist jedoch die Tatsache, dass an den Elektrodenoberflächen Hydrolyse stattfindet, wobei Sauerstoff an der positiven und Wasserstoff an der negativen Elektrode gebildet werden. Diese Gasbildung kann den Strompfad zwischen den beiden Elektroden unterbinden und somit die Elektrophorese behindern. Nicht modifizierte Elektroden erlauben deshalb nur eine begrenzte Elektrophoresezeit.

Dieses Problem kann dadurch überkommen werden, dass entweder die Elektroden modifiziert werden, um beispielsweise unterschiedliche elektrochemische Reaktionen an den Elektroden ablaufen zu lassen, oder dass die Elektroden im offenen Luftraum angeordnet werden, so dass das Gas entweichen kann, oder dass ein Gasspeicherraum vorgesehen wird, der dazu führt, dass sich das Gas in eine Richtung bewegt, in der es nicht den Strompfad behindert.

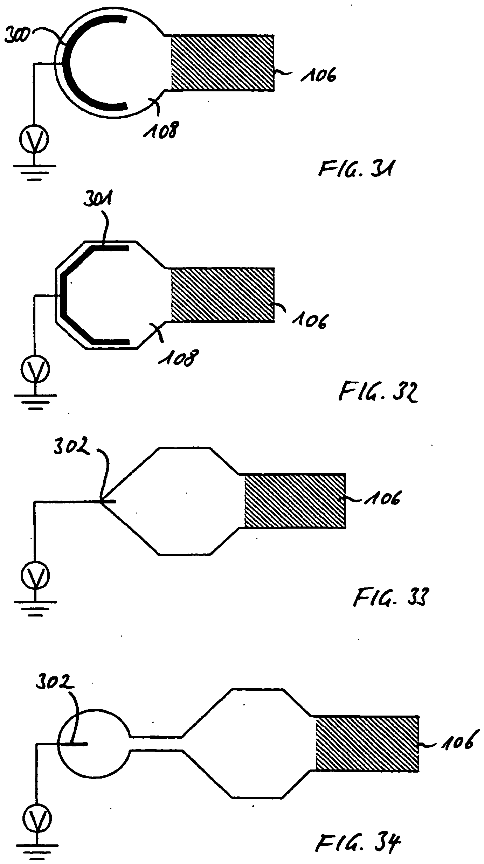

Mit Bezug auf die Figuren 31 bis 34, 35 bis 37 sowie 38 bis 40 werden verschiedene Elektrodenformen vorgeschlagen.

Die Figuren 31 bis 34 zeigen verschiedene integrierte Elektroden in einer geschlossenen Mikrokammer. Dabei sind die in den Figuren 31 und 32 gezeigten Elektroden am Umfang der Sammelkammer 108 angeordnet, so dass sie mit ihrer Form die Sammelkammer 108 begrenzen. Dabei sollte jeder Punkt der Elektrode 300 so strukturiert sein, dass er in etwa äquidistant zu dem Ausgang des Gelseparationspfades ist, um eine homogene Füllung der Sammelkammer zu gewährleisten. Analoges gilt für die Probenkammer, bei der es wünschenswert ist, dass über die ganze Probe hinweg ein homogenes elektrisches Feld auftritt. In den Figuren 33 und 34 sind Punktelektroden 302 skizziert.

Beispielsweise kann die Elektrode 302 in einer zusätzlichen Elektrodenkammer untergebracht sein (wie dies in Fig. 34 gezeigt ist) oder aber an einem äußersten Ende der Sammelkammer 108 angeordnet sein. Die Konfiguration der Figur 33 kann auch in einer Anordnung realisiert sein, bei der eine externe offene Elektrode vorgesehen ist.

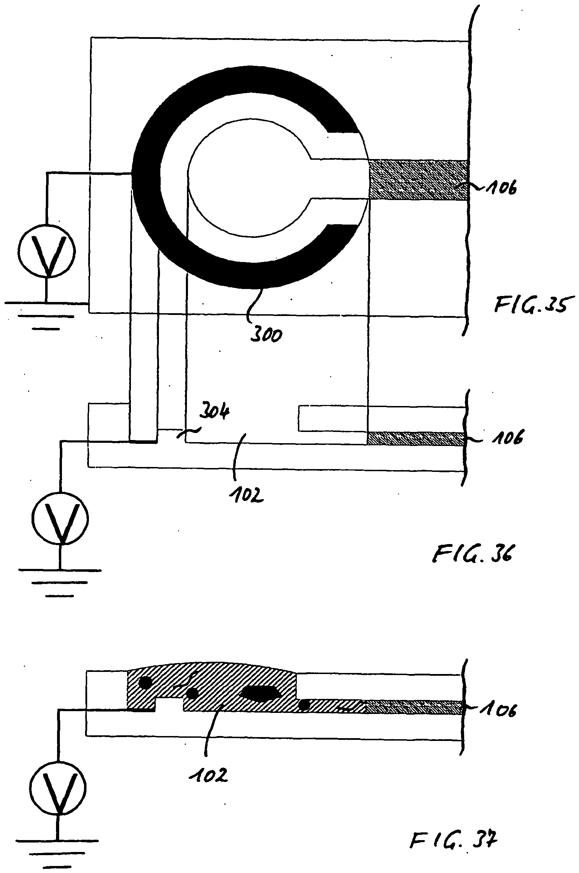

Eine solche offene Elektrode ist in Figuren 35 bis 37 dargestellt. Die Elektrode 300 ist hier der offenen Luft ausgesetzt, um nicht durch Gasbildung behindert zu werden. Die Elektroden sind in dieser gezeigten Ausführungsform in der Probenkammer ausgebildet. Die Probenkammer 102 enthält außerdem eine elektrisch isolierende Schwelle 304, die den Strom dazu zwingt, durch die Probe zu fließen, bevor er in den Gelseparationspfad eintritt. Die Probe ist hier als ein Tröpfchen von außen eingefüllt.

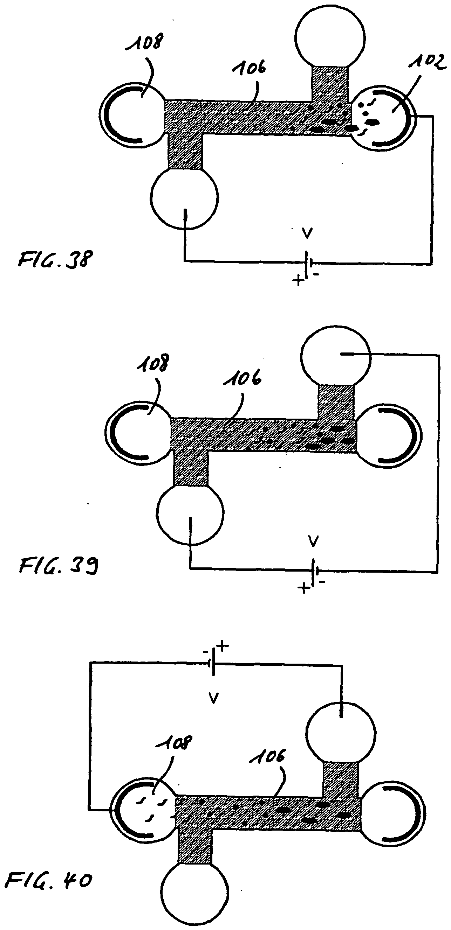

Wie in den Figuren 38 bis 40 skizziert, können aber auch integrierte und offene Elektroden kombiniert werden. In der hier gezeigten Ausführungsform wird eine erste Wanderung der Probe in das Gel dadurch angeregt, dass die Elektrode der Probenkammer zusammen mit einer offenen Elektrode in einem Seitenkanal aktuiert wird. Wenn die Probe vollständig in das Gel eingetreten ist, erfolgt die Separation durch ein Aktuieren der offenen Elektroden (Fig. 39). Das Eluieren des aufgereinigten Analyten schließlich erfolgt durch Aktuieren der integrierten Elektrode in einer Elutionskammer und einer offenen Elektrode an einer entfernten Stelle des Separationskanals. Die hier gezeigte Ausführungsform hat den Vorteil, dass die Separationszeiten nicht durch Gasbildung an den Elektroden behindert werden. Eine Verdampfung ist hier kein Problem, da die offenen Elektroden mit Puffer gespült werden können, ohne dadurch den Reinigungsprozess zu beeinflussen.

Weiterhin können in einer geschlossenen Kammer modifizierte Elektroden verwendet werden. Die Elektroden können dabei so modifiziert werden, dass eine Hydrolyse durch eine alternative elektrochemische Reaktion ersetzt wird. Beispielweise kann die negative Elektrode aus Silberchlorid gebildet werden, so dass während des Aktuatiosprozesses das Silberchlorid in Silber umgewandelt wird und dadurch Chloridionen entstehen. Mit einer solchen Anordnung kann die Wasserstofferzeugung an der negativen Elektrode verhindert werden. Andere Materialien, die für die negative Elektrode verwendet werden können, sind Silberiodid, Silberbromid, Zinksulfat und Kupfersulfat. Auch Palladium, das dafür bekannt ist, dass es Wasserstoff an seiner Oberfläche speichern kann, kann für die negative Elektrode verwendet werden, um eine Gasbildung zu verhindern.

Für die positive Elektrode kann beispielsweise Silber verwendet werden, das dann in eines der oben genannten Materialien übergeht, je nachdem welche Pufferbedingungen vorherrschen. Auf diese Weise kann die Erzeugung von Sauerstoff an der positiven Elektrode unterbunden werden.

Wie bereits erwähnt, hat ein dreidimensionales System mit vertikaler Komponente eine Anzahl signifikanter Vorteile gegenüber ausschließlich horizontalen flachen Systemen. Das Verhältnis der Geloberfläche gegenüber dem Probenvolumen kann leicht erhöht werden und zusätzlich kann eine sehr einfache Implementierung eines modularen Systems, in dem die empfindliche Affinitätskomponente getrennt von dem einfachen Vorbehandlungssystem hergestellt wird, realisiert-werden.

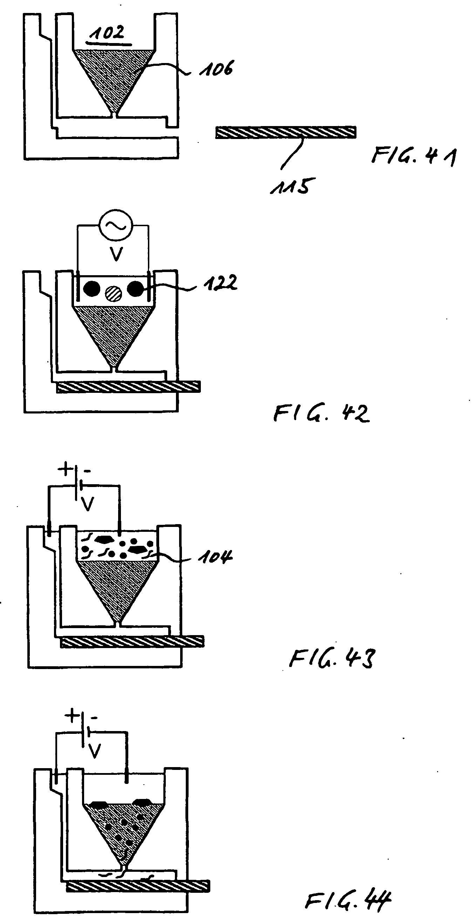

Ein Beispiel für eine solche modulare dreidimensionale Anordnung gemäß den Prinzipien der vorliegenden Erfindung ist in den Figuren 41 bis 44 gezeigt. Gemäß dieser Ausführungsform hat das Separationsgel eine konische Gestalt. Die Herstellung von Mehrfach-Gelseparationsstrecken kann ganz einfach durch eine geschichtete Abscheidung verschiedener Gelschichten erfolgen. Der Affinitätschip 115 wird von der Seite eingeschoben und schtießt so den mikrofluidischen Raum. In dem gezeigten Ausführungsbeispiel werden äußere offene Elektroden für die Aktuierung verwendet. Abhängig von der Zellaufschlussmethode können außerdem zusätzliche Elektroden für den Zellaufschluss vorgesehen sein. Der Aufschluss der Zellen ist hier in Figur 42 gezeigt, während die Fig. 43 und 44 den E-lektrophoresevorgang zeigen.

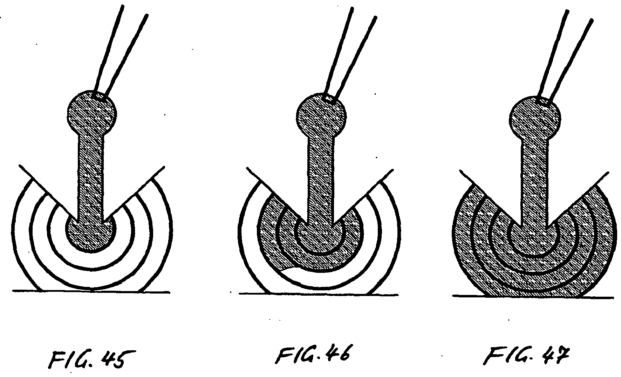

Mit Bezug auf die Figuren 45 bis 47 soll nachfolgend ein mögliches Herstellungsverfahren für die Gelseparationsstrecke beschrieben werden. Das Gel kann in ein Mikrosystem mittels manueller Injektion oder mit Hilfe einer Pumpe oder eines Mikrodosiersystems eingebracht werden. Typischerweise geschieht dies unter Verwendung einer Pipette. Um ein Gel mit einer reproduzierbaren Struktur herzustellen, können Phasenführungen, so genannte "Phaseguides", verwendet werden. Die Phasenführungen sind dünne Streifen eines Materials, das lokal die Kapillarkräfte in einem Fluidsystem ändert und somit Druckbarrieren aufbaut.

Figuren 45 bis 47 zeigen eine Gelstrukturierung mit Hilfe Phasenführungen, bei der das Gel mit einer Pipette eingebracht wird. Die Phasenführungen steuern das Fortschreiten der Gelfront, so dass das Gel in einer reproduzierbaren Struktur eingefüllt werden kann.

Mit Hilfe der erfindungsgemäßen integrierten mikrofluidischen Anordnung kann also eine hochsensisitive, automatisierte und miniaturisierte Aufreinigung und Detektion von Analytmolekülen durchgeführt werden. Insbesondere im Zusammenhang mit tmRNA-Molekülen kann damit ein Nachweis bakterieller Spezies ohne Verwendung von PCR-Schritten durchgeführt werden. In integrierter Form können Probenkonditionierung und Affinitätsdetektion innerhalb eines geschlossenen mikrofluidischen Bauteils durchgeführt werden.

## Patentansprüche

1. Integriertes mikro-fluidisches Bauteil zum Aufreinigen von Nukleinsäuremolekülen, wobei das Bauteil (100) umfasst mindestens eine Probenkammer (102) zum Aufnehmen einer die Nukleinsäuremoleküle enthaltenden Probe, wobei die Probe ein Zelllysat oder eine Zellenmischung ist; mindestens eine elektrophoretische Gelfiltrationsstrecke (106) zum Trennen der Nukleinsäuremoleküle von anderen Bestandteilen der Probe; mindestens eine Sammelkammer (108) zum Aufnehmen der aufgereinigten Nukleinsäuremoleküle , wobei das mikrofluidische Bauteil mindestens eine integrierte Rezeptoreinrichtung (118) zum Erfassen der Anwesenheit und/oder der Konzentration der aufgereinigten Nukleinsäuremoleküle aufweist, **dadurch gekennzeichnet, dass** die elektrophoretische Gelfiltrationsstrecke (106) unmittelbar mit der Probenkammer (102) verbunden ist, so dass die Probe unmittelbar durch die elektrophoretische Filtration aufgereinigt wird.

2. Integriertes fluidisches Bauteil nach Anspruch 1 , wobei die Rezeptoreinrichtung eine arrayförmig strukturierte Erkennungsschicht (115) zum selektiven Anbinden der aufgereinigten Ribonukleinsäuremoleküle umfasst.

3. Integriertes fluidisches Bauteil nach einem der Ansprüche 1 oder 2, wobei die Rezeptoreinrichtung eingerichtet ist, selektiv gebundene Ribonukleinsäuremoleküle optisch mittels Fluoreszenz oder elektrisch über einen Ladungseffekt, vorzugsweise impedanzspektroskopisch, potentiometrisch oder amperometrisch oder mittels Oberflächenplasmonenresonanz zu detektieren.

4. Integriertes fluidisches Bauteil nach mindestens einem der vorangegangenen Ansprüche, wobei die elektrophoretische Gelfiltrationsstrecke (106) durch eine Sequenz von Gelen oder einen Gradienten von Gelen gebildet ist, die in ihrer Porengrösse, ihrer chemischen Zusammensetzung und/oder ihrem pH-Wert variieren.

5. Integriertes fluidisches Bauteil nach mindestens einem der vorangegangenen Ansprüche, wobei das Gel über einen Seitenkanal mittels eines externen Dosiersystems, vorzugsweise einer Pipette, einbringbar ist.

6. Integriertes fluidisches Bauteil nach Anspruch 5, wobei das Gel durch eine räumliche Steuerung infolge der Anwesenheit von Phasenführungen strukturierbar ist.

7. Integriertes fluidisches Bauteil nach mindestens einem der vorangegangenen Ansprüche, wobei das Bauteil weiterhin mindestens eine integrierte Konditionierungsvorrichtung zum Behandeln der Probe und/oder zum Behandeln der aufgereinigten Ribonukleinsäuremoleküle umfasst, wobei die Konditionierungsvorrichtung zwischen eine Aufschlusseinrichtung zum Aufschliessen der Probe in der Probenkammer, eine Einrichtung zum Hybridisierungs-, Separations-, Filtrations-, Wash- und Fokussierungs-, Aufkonzentrations-, Transports-, Sedimentierungs-, Adsorptions-, oder Degradierungsschritte, und zum Real-time PCR oder Real-time NASBA (Nucleic Acid Sequence Base Amplification) einzuführen, selektiert ist.

8. Integriertes fluidisches Bauteil nach Anspruch 7, wobei die Konditionierungsvorrichtung eine Aufschlusseinrichtung zum Aufschliessen der Probe in der Probenkammer umfasst und wobei die Aufschlusseinrichtung betreibbar ist, eine Zelllyse, vorzugsweise eine elektrische, thermische, mechanische, ultraschallgestützte, osmotische, enzymatische und/oder chemische Zelllyse, durchzuführen.

9. Integriertes fluidisches Bauteil nach Anspruch 8, wobei die thermische Zelllyse durch Anlegen eines Wechselstroms erfolgt.

10. Integriertes fluidisches Bauteil nach mindestens einem der vorangegangenen Ansprüche, wobei die Rezeptoreinrichtung durch ein von dem übrigen Bauteil getrenntes Modul gebildet ist, das vor einer Messung mit dem Bauteil verbindbar ist.

11. Integriertes fluidisches Bauteil nach mindestens einem der vorangegangenen Ansprüche, wobei eine Bewegungsrichtung der Probe durch die Separationsstrecke quer zu einer Erkennungsschicht der Rezeptoreinheit verläuft.

12. Integriertes fluidisches Bauteil nach Anspruch 11, wobei die Separationsstrecke konisch geformt ist und die Probenkammer ein grösseres Volumen hat als die Sammelkammer.

13. Integriertes fluidisches Bauteil nach mindestens einem der vorgegangenen Ansprüche, wobei die Rezeptoreinrichtung betreibbar ist, ein Hybridisierungsexperiment durchzuführen.

14. Verfahren zum Aufreinigen von Ribonukleinsäuremolekülen mittels eines integrierten mikrofluidischen Bauteils der Ansprüche 1 bis 13, das die folgenden Schritte umfasst: Befüllen einer auf dem integrierten fluidischen Bauteil angeordneten Probenkammer mit einer die Ribonukleinsäuremoleküle enthaltenden Probe, wobei die Probe ein Zelllysat oder eine Zellenmischung ist; Transportieren der Probe entlang einer auf dem integrierten fluidischen Bauteil angeordneten elektrophoretischen Gelfiltrationsstrecke, wobei die elektrophoretische Gelfiltrationsstrecke unmittelbar mit der Probenkammer verbunden ist, so dass die Probe unmittelbar durch die elektrophoretische Filtration aufgereinigt wird, und wobei die Gelfiltrationsstrecke angepasst ist, die Ribonukleinsäuremoleküle von der Probe abzutrennen; Sammeln der aufgereinigten Ribonukleinsäuremoleküle in einer auf dem integrierten fluidischen Bauteil angeordneten Sammelkammer; Erfassen der Anwesenheit und/oder der Konzentration der aufgereinigten Ribonukleinsäuremoleküle mittels einer auf dem mikrofluidischen Bauteil integrierten Rezeptoreinrichtung.

15. Verfahren nach Anspruch 14, wobei einzelne Teile der Sequenz von Gelen durch Elektroden, die in der elektrophoretischen Gelfiltrationsstrecke integriert sind, unabhängig voneinander aktuierbar sind.

16. Verfahren nach einem der Ansprüche 14 bis 15, wobei die Zellenmischung nachzuweisende Viren und/oder Bakterien enthält.

## Claims

1. Integrated microfluidic component for purifying nucleic acid molecules, where the component (100) comprises at least one sample chamber (102) for receiving a sample containing the nucleic acid molecules, where the sample is a cell lysate or a cell mixture; at least one electrophoretic gel filtration track (106) for separating the nucleic acid molecules from other constituents of the sample; at least one collection chamber (108) for receiving the purified nucleic acid molecules, where the microfluidic component has at least one integrated receptor device (114, 115) for determining the presence and/or the concentration of the purified nucleic acid molecules, **characterised in that** the electrophoretic gel filtration track (106) is connected directly to the sample chamber (102), such that the sample is purified directly by electrophoretic filtration.

2. Integrated fluidic component according to Claim 1, where the receptor device comprises an array-like structured recognition layer (114) for the selective binding of the purified ribonucleic acid molecules.

3. Integrated fluidic component according to one of Claims 1 or 2, where the receptor device is equipped to detect selectively bound ribonucleic acid molecules optically by means of fluorescence or electrically by means of a charge effect, preferably by impedance spectroscopy, potentiometrically or amperometrically or by means of surface plasmon resonance.

4. Integrated fluidic component according to at least one of the preceding claims, where the electrophoretic gel filtration track (106) is formed by a sequence of gels or a gradient of gels that vary in their pore size, their chemical composition and/or their pH.

5. Integrated fluidic component according to at least one of the preceding claims, where the gel is introducible via a side channel by means of an external metering system, preferably a pipette.

6. Integrated fluidic components according to Claim 5, where the gel is structurable by a spatial control owing to the presence of phaseguides.

7. Integrated fluidic component according to at least one of the preceding claims, where the component comprises at least one integrated conditioning device for treating the sample and/or for treating the purified ribonucleic acid molecules, where the conditioning device to be introduced is selected from amongst a digestion device for digesting the sample in the sample chamber, a device for hybridisation, separation, filtration, washing and focusing, concentration, transport, sedimentation, adsorption or degradation steps, and for real-time PCR or real-time NASBA (nucleic acid sequence base amplification).

8. Integrated fluidic component according to Claim 7, where the conditioning device comprises a digestion device for digesting the sample in the sample chamber and where the digestion device is operable for carrying out a cell lysis, preferably an electrical, thermal, mechanical, ultrasound-based, osmotic, enzymatic and/or chemical cell lysis.

9. Integrated fluidic component according to Claim 8, where the thermal cell lysis is carried out by applying an alternating current.

10. Integrated fluidic component according to at least one of the preceding claims, where the receptor device is formed by a module separate from the other component, which can be connected to the component before a measurement.

11. Integrated fluidic component according to at least one of the preceding claims, where one direction of motion of the sample runs through the separation track at right angles to a recognition layer of the receptor unit.

12. Integrated fluidic component according to Claim 11, where the separation track is conically shaped and the sample chamber has a larger volume than the collection chamber.

13. Integrated fluidic component according to at least one of the preceding claims, where the receptor device is operable for carrying out a hybridisation experiment.

14. Process for purifying ribonucleic acid molecules by means of an integrated microfluidic component of Claims 1 to 13, which comprises the following steps: filling of a sample chamber arranged on the integrated fluidic component with a sample containing the ribonucleic acid molecules, where the sample is a cell lysate or a cell mixture; transportation of the sample along an electrophoretic gel filtration track arranged on the integrated fluidic component, where the electrophoretic gel filtration track is connected directly to the sample chamber such that the sample is purified directly by electrophoretic filtration, and where the gel filtration track is adapted to separate the ribonucleic acid molecules from the sample; collection of the purified ribonucleic acid molecules in a collection chamber arranged on the integrated fluidic component; and detection of the presence and/or the concentration of the purified ribonucleic acid molecules by means of a receptor device integrated on the microfluidic component.

15. Process according to Claim 14, where individual parts of the sequence of gels are actuatable independently of one another by electrodes that are integrated into the electrophoretic gel filtration track.

16. Process according to one of Claims 14 to 15, where the cell mixture contains viruses and/or bacteria to be detected.

## Revendications

1. Composant microfluidique intégré pour la purification de molécules d'acide nucléique, où le composant (100) comprend au moins une chambre à échantillons (102) destinée à accueillir un échantillon contenant les molécules d'acide nucléique, où l'échantillon est un lysat cellulaire ou un mélange de cellules ; au moins une portion de filtration sur gel par électrophorèse (106) destinée à séparer les molécules d'acide nucléique des autres constituants de l'échantillon ; au moins une chambre de collecte (108) destinée à recueillir les molécules d'acide nucléique purifiées, où le composant microfluidique comprend au moins un dispositif récepteur intégré (114, 115) pour détecter la présence et/ou la concentration des molécules d'acide nucléique purifiées, **caractérisé en ce que** la portion de filtration sur gel par électrophorèse (106) est directement reliée à la chambre à échantillons (102) afin que l'échantillon soit purifié directement par filtration par électrophorèse.

2. Composant fluidique intégré selon la revendication 1, où le dispositif récepteur comprend une couche d'identification (114) structurée en forme de réseau pour la liaison sélective des molécules d'acide ribonucléique purifiées.

3. Composant fluidique intégré selon l'une des revendications 1 ou 2, où le dispositif récepteur est aménagé afin de détecter les molécules d'acide ribonucléique sélectivement liées optiquement au moyen de la fluorescence ou bien électriquement par un effet de charge, de préférence par spectroscopie d'impédance, potentiométrie ou ampérométrie, ou au moyen de la résonance plasmonique de surface.

4. Composant fluidique intégré selon au moins l'une des revendications précédentes, où la portion de filtration sur gel par électrophorèse (106) est formée par une séquence de gels ou un gradient de gels, qui varient au niveau de leur taille de pores, leur composition chimique et/ou leur pH.

5. Composant fluidique intégré selon au moins l'une des revendications précédentes, où le gel peut être introduit par le biais d'un canal latéral, au moyen d'un système de dosage externe, de préférence une pipette.

6. Composant fluidique intégré selon la revendication 5, où le gel peut être structuré par une commande spatiale à la suite de la présence de guidages de phases.

7. Composant fluidique intégré selon au moins l'une des revendications précédentes, où le composant comprend également au moins un dispositif de conditionnement intégré, destiné au traitement de l'échantillon et/ou au traitement des molécules d'acide ribonucléique purifiées, où le dispositif de conditionnement est choisi parmi un système de désintégration pour désintégrer l'échantillon dans la chambre à échantillons, un système destiné à la réalisation d'étapes d'hybridation, de séparation, de filtration, de lavage et de focalisation, de reconcentration, de transport, de sédimentation, d'adsorption ou de dégradation et d'une PCR en temps réel ou d'une NASBA (Nucleic Acid Sequence Base Amplification) en temps réel.

8. Composant fluidique intégré selon la revendication 7, où le dispositif de conditionnement comprend un système de désintégration pour désintégrer l'échantillon dans la chambre à échantillons et où le système de désintégration est exploitable dans le but de réaliser une lyse cellulaire, de préférence une lyse cellulaire électrique, thermique, mécanique, assistée par ultrasons, osmotique, enzymatique et/ou chimique.

9. Composant fluidique intégré selon la revendication 8, où la lyse cellulaire thermique s'effectue par application d'un courant alternatif.

10. Composant fluidique intégré selon au moins l'une des revendications précédentes, où le dispositif récepteur est formé par un module séparé du reste du composant, qui peut être relié au composant avant une mesure.

11. Composant fluidique intégré selon au moins l'une des revendications précédentes, où une direction de mouvement de l'échantillon à travers la portion de séparation est oblique par rapport à une couche d'identification de l'unité du récepteur.

12. Composant fluidique intégré selon la revendication 11, où la portion de séparation a une forme conique et la chambre à échantillons a un volume plus important que la chambre de collecte.

13. Composant fluidique intégré selon au moins l'une des revendications précédentes, où le dispositif récepteur est exploitable dans le but de réaliser une expérience d'hybridation.

14. Procédé de purification de molécules d'acide ribonucléique au moyen d'un composant microfluidique intégré selon les revendications 1 à 13, qui comprend les étapes suivantes : remplissage d'une chambre à échantillons disposée sur le composant fluidique intégré avec un échantillon contenant les molécules d'acide ribonucléique, où l'échantillon est un lysat cellulaire ou un mélange de cellules ; transport de l'échantillon le long d'une portion de filtration sur gel par électrophorèse disposée sur le composant fluidique intégré, où la portion de filtration sur gel par électrophorèse est reliée directement à la chambre à échantillons afin que l'échantillon soit purifié directement par filtration par électrophorèse, et où la portion de filtration sur gel est adaptée à séparer les molécules d'acide ribonucléique et l'échantillon ; collecte des molécules d'acide ribonucléique purifiées dans une chambre de collecte disposée sur le composant fluidique intégré ; détection de la présence et/ou de la concentration des molécules d'acide ribonucléique purifiées au moyen d'un dispositif récepteur intégré sur le composant microfluidique.

15. Procédé selon la revendication 14, où les différentes parties de la séquence de gels sont actionnables indépendamment les unes des autres grâce à des électrodes, qui sont intégrées à la portion de filtration sur gel par électrophorèse.

16. Procédé selon l'une des revendications 14 à 15, où le mélange de cellules contient des virus et/ou des bactéries qui sont à détecter.
